# EUROPEAN PATENT APPLICATION

(11) **EP 2 425 838 A2**
(43) Date of publication of application: **07.03.2012**
(21) Application number: 11173881.1
(22) Date of filing: 26.02.2008
(51) Int. Cl.: A61K 31/55, A61K 39/395, A61P 35/00, A61P 37/00, A61K 31/5575, A61K 38/19, A61K 38/20, A61K 45/06, C07K 14/705, C07K 14/715, C07K 16/24

(54) **Combination therapy for treatment of immune disorders**

(30) Priority: 28.02.2007 US 892142 P; 20.06.2007 US 945279 P
(62) Divisional of application: 08714219.6
(71) Applicant: Schering Corporation, Kenilworth, NJ 07033-0530 (US)
(72) Inventor: Bowman, Edward, Paul, Redwood City,, CA California 94061 (US); Cua, Daniel J., Boulder Creed,, CA California 95006 (US); Kastelein, Robert, A., Portola Valley,, CA California 94028 (US); Miller, Kathy, Lynn, San Francisco,, CA California 94133 (US); Kleinschek, Melanie, A., San Francisco,, CA California 94110 (US); Bak-Jensen, Kristian, Sass, DK-1722 Copenhagen V. (DK); Boniface, Katia,, Mountain View,, CA California 94040 (US); McKenzie, Brent, S., Victoria,, Victoria DK-1722 (AU); De Waal Malefyt, Rene,, Sunnyvale,, CA California 94086 (US)
(74) Representative: Zvesper, Thomas

(57) **Abstract**

Methods and compositions are provided for the treatment of immune disorders, such as autoimmune diseases, or cancers, involving combination therapy with agents that inhibit the development or maintenance of Th17 cells. Treatment regimens are provided in which an antagonist of a pro-inflammatory cytokine is administered for a time sufficient to alleviate signs and symptoms of an acute phase flare-up of the autoimmune disease, or cancer, and treatment with an antagonist of IL-23 is continued for a longer time to prevent recurrence of the acute event. Antagonists of PGE2 and CD 161 are also disclosed for use in treatment of autoimmune, inflammatory and proliferative disorders.

## Description

### FIELD OF THE INVENTION

The present invention relates compositions and methods for treatment of immune disorders, such as autoimmune disorders. Specifically, the invention relates to combination therapy with agents that inhibit the development or maintenance of Th17 cells.

### BACKGROUND OF THE INVENTION

The immune system functions to protect individuals from infective agents, e.g., bacteria, multi-cellular organisms, and viruses, as well as from cancers. This system includes several types of lymphoid and myeloid cells such as monocytes, macrophages, dendritic cells (DCs), eosinophils, T cells, B cells, and neutrophils. These lymphoid and myeloid cells often produce signaling proteins known as cytokines. The immune response includes inflammation, i.e., the accumulation of immune cells systemically or in a particular location of the body. In response to an infective agent or foreign substance, immune cells secrete cytokines which, in turn, modulate immune cell proliferation, development, differentiation, or migration. Immune response can produce pathological consequences, e.g., when it involves excessive inflammation, as in the autoimmune disorders. *See, e.g.,* Abbas et al. (eds.) (2000) Cellular and Molecular Immunology, W.B. Saunders Co., Philadelphia, PA; Oppenheim and Feldmann (eds.) (2001) Cytokine Reference, Academic Press, San Diego, CA; von Andrian and Mackay (2000) New Engl. J. Med. 343:1020-1034; Davidson and Diamond (2001) New Engl. J Med. 345:340-350).

Many cytokines have been implicated in diseases involving aberrant inflammatory responses.

IL-17, which was originally named cytotoxic T-Lymphocyte-associated antigen 8 (CTLA8) is a homodimeric cytokine that binds to IL-17RA (also known as IL17R) and IL-17RC. The functional receptor for IL-17 is bel ieved to be a multimeric receptor complex comprising one or both of IL-17RA and IL-17RC (e.g., an IL-17RA homodimer, an IL-17RC homodimer, or an IL-17RA/IL-17RC heterodimer) and possibly a third, as yet unknown, protein (Toy et al. (2006) J. Immunol. 177(1):36-39; unpublished data).

IL-17 activity (reviewed in Kolls et al. (2004) Immunity 21:467-476) includes promoting accumulation of neutrophils in a localized area and the activation of neutrophils. IL-17 can induce or promote the production of any of the following proinflammatory and neutrophil-mobilizing cytokines, depending on the cell type: IL-6, MCP-1, CXCL8 (IL-8), CXCL1, CXCL6, TNFα, IL-1β, G-CSF, GM-CSF, MMP-1, and MMP-13.

Interleukin-12 (IL-12) is a heterodimeric molecule composed of p35 and p40 subunits. Studies have indicated that IL-12 plays a critical role in the differentiation of naïve T cells into T-helper type 1 CD4⁺ lymphocytes that secrete IFNγ. It has also been shown that IL-12 is essential for T cell dependent immune and inflammatory responses in *vivo. See, e.g.,* Cua et al. (2003) Nature 421:744-748. IL-12 receptor is a complex of IL-12Rβ1 and IL-12Rβ2 subunits. *See* Presky et al. (1996) Proc. Nat'l Acad. Sci. USA 93:14002.

Interleukin-23 (IL-23) is a heterodimeric cytokine comprised of two subunits, p19 which is unique to IL-23, and p40, which is shared with IL-12. The p19 subunit is structurally related to IL-6, granulocyte-colony stimulating factor (G-CSF), and the p35 subunit of IL-12. IL-23 mediates signaling by binding to a heterodimeric receptor, comprised of IL-23R, which is unique to IL-23 receptor, and IL-12Rβ1, which is shared by the IL-12 receptor. *See* Parham et al. (2000) J. Immunol. 168:5699.

IL-23 activity includes inducing the proliferation of memory T cells, PHA blasts, CD45RO T cells; and enhance production of interferon-gamma (IFNγ) by PHA blasts or CD45RO T cells. In contrast to IL-12, IL-23 preferentially stimulates memory as opposed to naïve T cell populations in both human and mouse. IL-23 activates a number of intracellular cell-signaling molecules, e.g., Jak2, Tyk2, Stat1, Stat2, Stat3, and Stat4. IL-12 activates this same group of molecules, but Stat4 response to IL-23 is relatively weak, while Stat4 response to IL-12 is strong. Oppmann et al. (2000) Immunity 13:715-725; Parham et al. (2002) J Immunol. 168:5699-5708. IL-23 has also been implicated in the maintenance and proliferation ofIL-17 producing cells, also known as Th17 cells. *See* Cua and Kastelein (2006) Nature Immunology 7:557 ― 559.

Comparison of the natural roles of IL-12 and IL-23 suggest that targeting IL-23 for inhibition will cause fewer adverse side-effects when compared with inhibition of IL-12, or inhibition of both IL-23 and IL-12. Bowman et al. (2006) Curr. Opin. Infect. Dis. 19:245. While IL-12 is critical to mounting a systemic Th1-mediated immune responses, IL-23 (along with IL-1β, IL-6 and TNF-α) is thought to be responsible for promotion and maintenance of Th-17 cells. Such Th17 cells are believed to be involved in responses to catastrophic injury, such as breach of the mucosal barrier of the lung or gut, and the resulting exposure to the deadly pathogens *K. pneumoniae* and C. *rodentium.* Such catastrophic injuries would almost certainly require an immediate immune response in the form of massive neutrophil influx. *See* Cua and Kastelein (2006) Nature Immunology 7:557. Because such catastrophic injuries and infections are relatively rare in modern society, and can be treated with antibiotics if they do occur, this Th17 "nuclear option" may not be as critical to survival as it was earlier in human evolution. This suggests that disruption of IL-23 / IL-23 receptor signaling may have a relatively minor side effect profile, since its natural activity is of little importance in modem society. *See* McKenzie et al. (2006) Trends Immunol. 27:17.

The distinct subunit compositions of IL-12 receptor and IL-23 receptor make it possible to design therapy that targets only IL-23 receptor but not IL-12 receptor. Compounds that bind to and inhibit the activity of IL-23p19 or IL-23R, either in isolation of as components of their respective heterodimeric complexes, will inhibit IL-23 but not IL-12. There may also be compounds that are capable of binding to IL-12p40 when present in IL-23 but not in IL-12, or compounds that bind to and inhibit IL-12Rβ1 when present in the IL-23 receptor but not in IL-12 receptor. Such specific binding agents will also inhibit IL-23 activity but not IL-12 activity. IL-23/IL-23R specific agents would be expected to be safer (i.e. have a lower side effect profile) than agents that also inhibit IL-12.

Much of the early work on inhibition of IL-12 involved inhibition of IL-12p40. It has been subsequently realized that these experiments involved not only inhibition ofIL-12 but also inhibition of IL-23, and that in fact the effects in many of these experiments were the result of inhibition of IL-23. Many disorders once thought to be caused by a pathogenic Th1 response, which could be ameliorated by inhibition of IL-12, have been shown instead to be caused by a Th17 response, which is ameliorated by inhibition of IL-23. Yen et al. (2006) J. Clin. Invest. 116:1310; Iwakura and Ishingame (2006) J. Clin. Invest. 116:1218.

IL-23R has been implicated as a critical genetic factor in the inflammatory bowel disorders, Crohn's disease and ulcerative colitis. Duerr et al. (2006) Sciencexpress 26-October-2006:1. A genome-wide association study found that the gene for IL-23R was highly associated with Crohn's disease, with an uncommon coding variant (Arg381Gln) conferring strong protection against the disease. This genetic association confirms prior biological findings (Yen et al. (2006) J. Clin. Investigation 116:1218) suggesting that IL-23 and its receptor are promising targets for new therapeutic approached to treating IBD.

Recent findings have demonstrated that IL-23 is important in promoting the survival and proliferation of a class ofT cells referred to as Th17 cells. A recent paper reported that IL-23 promotes a T cell population characterized by the production ofIL-17, IL-1 7F, TNF, IL-6 and other factors, referred to as "Th17 cells" (Langrish et al. (2005) J. Exp. Med. 201:233-240). Production of such Th17 cells is promoted by IL-6 and TGF-β. *See, e.g.,* Veldhoen et al. (2006) Immunity 24:179-189; Dong (2006) Nat. Rev. Immunol. 6(4):329-333. IL-22 has also been proposed as an important Th17 cytokine. *See, e.g.,* U.S. Patent Application Publication No. 2008/0031882A1. Based on current understanding, IL-23 is responsible for maintenance and proliferation of this new class of helper T cells, although it is not necessary for the initial creation of Th17 cells.

A number of autoimmune diseases are known to involve periods of acute "flare-up" of signs and symptoms, followed by extended periods that are relatively asymptomatic. Such diseases are sometimes referred to as "relapsing-remitting" diseases. The prototypical relapsing-remitting disease is multiple sclerosis (MS), in which 85% of subjects suffer from the relapsing-remitting form of the disease, as opposed to a progressive form of the disease. Relapsing-remitting MS is characterized by clearly defined acute attacks followed by periods of full recovery, or stabilization with some deficit. Primary symptoms of MS include fatigue (also called MS lassitude to differentiate it from tiredness resulting from other causes), problems with walking, bowel and or bladder disturbances, visual problems, changes in cognitive function (including problems with memory, attention, and problem-solving), abnormal sensations (such as numbness or "pins and needles"), changes in sexual function, pain, depression and/or mood swings, and less frequently, tremor, incoordination, speech and swallowing problems and impaired hearing. Current pharmaceutical interventions include interferon beta la (IFN-β1a), interferon beta 1b (IFN-β1b), and the humanized anti-integrin-α4 antibody natalizumab.

Relapsing-remitting autoimmune diseases also include auto-inflammatory disorders, such as various hereditary periodic fever syndromes, Crohn's disease, Blau syndrome, Bechet's disease and systemic lupus erythematosus. Church et al. (2006) Springer Semin. Immun. 27:494. Biologic agents for treatment of hereditary periodic fever syndromes include antagonists of tumor necrosis factor alpha (TNF-α), such as infliximab, etanercept and adalimumab, and antagonists of interleukin-1 beta (IL-1β), such as the IL-1 receptor antagonist anakinra (Kineret^{®} IL-1 receptor antagonist).

The need exists for improved methods and compositions for the treatment of immune disorders, such as autoimmune disease. Preferably such methods and compositions would treat the acute symptoms of the disease, e.g. by alleviating the signs and symptoms of the disorder, and also reduce the likelihood of recurrence of the disease. Preferably such methods and compositions would comprise a comprehensive disease management protocol in which two or more therapeutic agents are administered in a therapeutic regimen that promotes rapid resolution of signs and symptoms, while also promoting long-term disease suppression.

### SUMMARY OF THE INVENTION

The present invention meets these needs in the art and more by providing compositions for treatment of immune disorders, such as autoimmune diseases, comprising an antagonist ofIL-23 and an antagonist of one or more pro-inflammatory cytokines, e.g. IL-17A, IL-17F, IL-1β and TNF-α. As used herein, IL-17A, IL-17F, IL-1β and TNF-α are referred to collectively as "acute phase cytokines," and antagonists of these cytokines are referred to collectively as "acute phase therapeutic agents". In one embodiment the subject is experiencing a flare-up of symptoms of the immune disorder at the start of treatment with the methods and compositions of the present invention.

In one aspect, the invention relates to methods of treatment of subjects having immune disorders, such as autoimmune diseases, comprising administering to said subject an effective amount of an antagonist of IL-23 and an antagonist of a pro-inflammatory cytokine selected from the group consisting of IL-17A, IL-17F, IL-1β and TNF-α. Such administration of two or more antagonists is referred to herein as combination therapy.

In one embodiment, one or more of the antagonists binds to a cytokine itself (e.g. IL-23, IL-17A, IL-17F, IL-1β or TNF-a), rather than its receptor. In another embodiment, one or more of the antagonists binds to a cytokine receptor.

In one embodiment the immune disorder is dysregulation of the Th17 response, giving rise to suppression of IL-12-mediated Th1 tumor surveillance. In such embodiments the methods and compositions of the present invention are used to treat subject with cancer or tumors.

In one embodiment, one or more antagonist of the present invention is an antibody or antigen binding fragment thereof. In various embodiments the antibody is a chimeric, humanized or fully human antibody, and the antigen binding fragment is a fragment of a chimeric, humanized or fully human antibody. In various embodiments the fragment is selected from the group consisting Fab, Fab', Fab'-SH, Fv, scFv, F(ab')₂, and a diabody. In one embodiment the antibody of antigen binding fragment thereof is PEGylated.

In various embodiments the IL-23 antagonist is an antagonist antibody, or antigen binding fragment thereof, that binds to IL-23p19 or IL-23R.

In one embodiment, the acute phase therapeutic agent is an antibody that specifically binds to a cytokine selected from the group consisting of IL-1β, TNF-α, IL-17A, and IL-17F. In another embodiment, the acute phase therapeutic agent is an antibody that specifically binds to a receptor for a cytokine selected from the group consisting of IL-1β, TNF-α, IL-17A, and IL-17F.

In another embodiment the antibody or antigen binding fragment thereof is a bispecific antibody or antigen binding fragment thereof. In various embodiments the bispecific antibody binds to and antagonizes IL-23 (e.g. IL-23p19) or IL-23 receptor (e.g. IL-23R), and also binds to and antagonizes an acute phase cytokine or a receptor of an acute phase cytokine.

In one embodiment, the invention relates to a bispecific antibody, or antigen binding fragment thereof, that binds to IL-23R and CD161. Other embodiments include bispecific reagents directed to CD 161 and at least one of CD4, CD45RO, CCR4, CCR6, integrin-β7, EP2, EP4, IL-1R1, or TNF-α. In various embodiments the bispecific antibody further comprises an IgG1 constant domain and/or a toxic payload, such as a radionuclide or other toxin.

In one embodiment, the invention relates to combination therapy using a bispecific reagent comprising a first polypeptide and a second polypeptide, wherein the first polypeptide comprises IL-1ra or a soluble TNF-α receptor fragment, and the second polypeptide comprises an antigen-binding fragment of an antibody that binds to IL-23, IL-23R, IL-17A, IL-17RA or IL-17RC. In one embodiment, each of the first and second polypeptides is fused to an antibody Fc domain.

In another embodiment, the invention relates to use of a combination of two or more agents selected from the group consisting of IL-23, IL-1β, and PGE2, or PGE2 alone, or agonists thereof, for the *in vitro* generation of pathogenic mammalian Th17 cells, e.g. mammals such as a mouse or a human. In some embodiments, T cells (e.g. naïve CD4⁺ T cells) are cultured in the presence of two or more agents selected from the group consisting of IL-23, IL-1β, and PGE2, e.g. PGE2 plus either IL-23 or IL-1β, or in the presence of PGE2 alone. In a further embodiment the invention relates to a method of screening for compounds for use in the treatment of disorders mediated by Th17 cells comprising generating pathogenic Th17 cells in *vitro* by culturing T cells (e.g. naïve CD4⁺ T cells) in the presence of two or more agents selected from the group consisting of IL-23, IL-1β, and PGE2, exposing said cells to one or more potential therapeutic compounds, and evaluating the effect of such compound(s) on said Th17 cells. In one embodiment said evaluating is by measurement of the level of expression of two or more cytokines selected from the group consisting of IL-17A, IL-17F, IL-10, IL-22 and IFN-γ. Compounds that inhibit the development or maintenance of Th17 cells, e.g. by lowering the expression of IL-17A or IL-17F, would be considered potential therapeutic agents.

In yet another embodiment, the invention relates to use of a combination of two or more agents selected from the group consisting of antagonists of IL-23, IL-1β, and PGE2, including antagonists of any of their respective receptors or receptor subunits, for the treatment of autoimmune or proliferative disorders. In one embodiment, the two or more agents are present as a single reagent, such as a bifunctional reagent (e.g. a protein) or a bispecific antibody (or antigen binding fragment thereof). In some embodiments, the autoimmune or proliferative disorder is caused by pathogenic Th17 cells. In some embodiments the agents are administered locally at (or near) the site of inflammation, whereas in other embodiments the agents are administered systemically, such as orally or parenterally.

In another embodiment, the invention relates to compositions comprising a combination of two or more agents selected from the group consisting of antagonists of IL-23, IL-1β, and PGE2, including antagonists of any of their respective receptors or receptor subunits, for use in the treatment of autoimmune or proliferative disorders. In some embodiments the antagonist of PGE2 is a cyclooxygenase (COX) inhibitor. In other embodiments the antagonist of PGE2 is a specific inhibitor of a PGE2 synthase. In some embodiments, the composition comprises a bifunctional reagent (e.g. a protein) or a bispecific antibody (or antigen binding fragment thereof).

In another embodiment, the invention relates to methods of treatment of autoimmune or proliferative disorders comprising the steps of (optionally) detecting the level of pathogenic Th17 cells in a subject (e.g. in a bodily fluid or tissue sample), administering a composition of the present invention to said subject, and (optionally) monitoring the level of said pathogenic Tb17 cells during and/or after administration of the composition to determine whether treatment is effective. In one embodiment the composition of the present invention comprises a combination of two or more agents selected from the group consisting of antagonists of IL-23, IL-1β, and PGE2, including antagonists of any of their respective receptors or receptor subunits, wherein such antagonists optionally comprise a bifunctional reagent (e.g. a protein) or a bispecific antibody (or antigen binding fragment thereof). In one embodiment said monitoring is by measurement of the level of expression of one, two, three or more cytokines selected from the group consisting of IL-17A, IL-17F, IL-10, IL-22 and IFN-γ. In one embodiment treatment is considered effective if said monitoring reveals reduced expression of Th 17 cytokines, e.g. IL-17A or IL-17F.

In one embodiment, the methods of the present invention further comprise administration of an immunosuppressive or anti-inflammatory agent, such as steroids (e.g. predinisone) and non-steroidal anti-inflammatory agents.

In various embodiments treatment with the IL-23 antagonist is continued as a series of one or more doses over a first time interval, and treatment with the acute phase therapeutic agent is continued as a series of one or more doses over a second time interval. In various embodiments the first time interval beings at substantially at the same time as the second time interval, sometime later during the second time interval, or after the end of the second time interval. In one embodiment, the first time interval extends beyond the end of the second time interval, i.e. IL-23 antagonist therapy continues after the cessation of treatment with the acute phase therapeutic agent. In various embodiments, the second time interval ends upon the resolution of at least one, two or more symptoms of the flare-up of the autoimmune disease.

In various embodiments the first and second time intervals are selected from the group consisting of 0.5, 1, 2, 3, 4, 5, 6, 7, 8, 9, 10, 12, 15, 18, 24, 36, 48, 60 or more months.

In various embodiments, the subject treated with the methods or compositions of the present invention has a disorder selected from the group consisting of cancer, arthritis, rheumatoid arthritis (RA), psoriasis, inflammatory bowel disease, Crohn's disease, ulcerative colitis, multiple sclerosis (MS), systemic lupus erythematosus (SLE), type I diabetes.

In another aspect, the invention relates to pharmaceutical compositions comprising an antagonist of IL-23 and an antagonist of an acute phase cytokine, e.g. IL-1β, TNF-α, IL-17A, or IL-17F. In one embodiment the pharmaceutical composition further comprises a pharmaceutically acceptable carrier or diluent. In another embodiment the pharmaceutical compositions of the present invention further comprise an immunosuppressive or anti-inflammatory agent, such as steroids (e.g. prednisone) and non-steroidal anti-inflammatory agents.

In another aspect, the invention relates to use of an antagonist of IL-23 and an antagonist of an acute phase cytokine in the manufacture of a medicament for the treatment of an immune disorder, such as cancer, arthritis, RA, psoriasis, inflammatory bowel disease, Crohn's disease, ulcerative colitis, MS, SLE, type I diabetes.

### BRIEF DESCRIPTION OF THE DRAWINGS

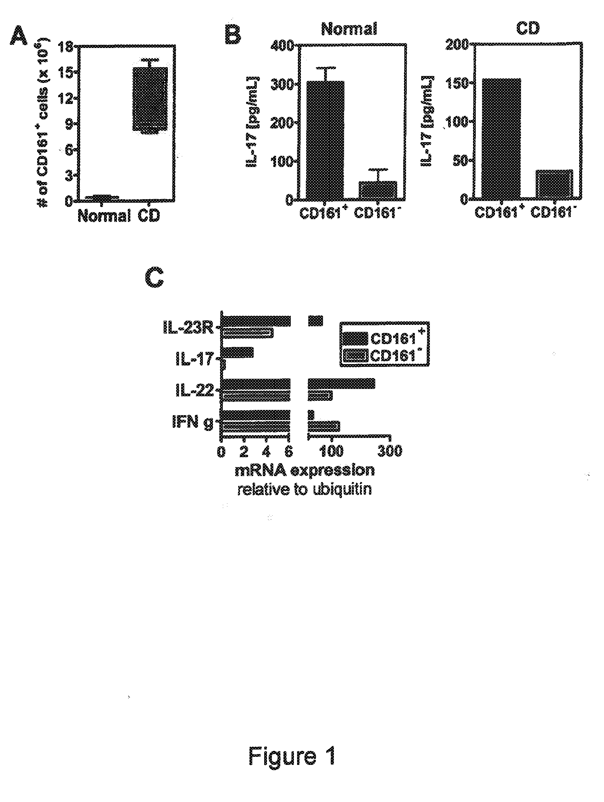

FIG. 1A shows the number of CD161⁺ CD4⁺ CD45RO⁺ T cells cells in lamina propria mononuclear cells (LPMC) from normal human subjects and from Crohn's disease patients. FIG. 1B shows IL-17A expression, as measured by enzyme-linked immunosorbent assay (ELISA), in fluorescence activated cell sorting (FACS^{®}) purified CD161⁺ and CD161⁻ CD4⁺ CD45RO⁺ T cells from normal human subjects and from Crohn's disease patients after three day culture with anti-CD2, anti-CD3, anti-CD28 activation beads. FIG. 1C shows relative mRNA expression levels for IL-23R. IL-17A, IL-22 and IFN-γ, as assessed by quantitative real time reverse transcriptase polymerase chain reaction (qRT-PCR), in mononuclear cells isolated from Crohn's disease human colon sorted for CD161⁺ and CD161⁻ cells within the CD4⁺ CD45RO⁺ memory T cells (Thₘₑₘ). Cells are sorted for CD161 expression by FACS^{®} flow cytometry.

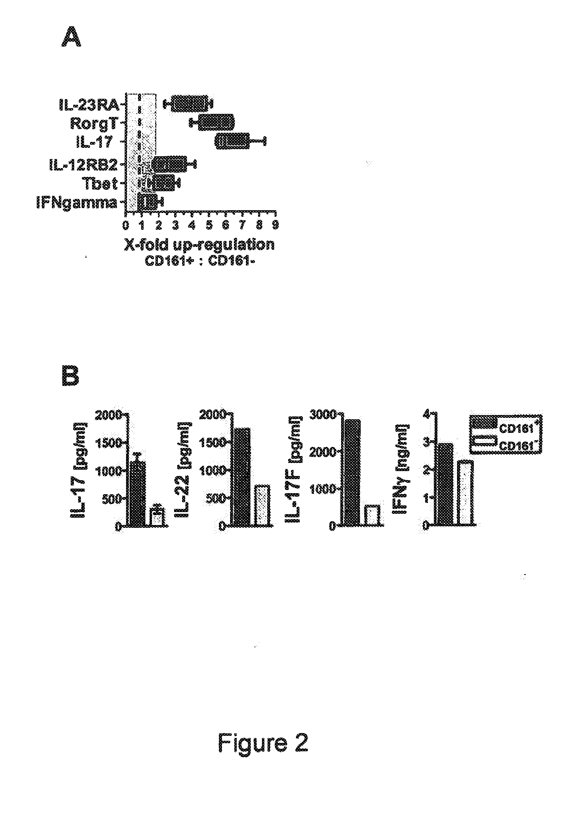

FIGS. 2A and 2B show gene expression, and cytokine production, respectively, in CD4⁺ CD25⁻ CD45RA⁻ memory T cells as a function of CD 161 expression for peripheral blood mononuclear cells (PBMC) from healthy human donors. Cells are sorted for CD161 expression by FACS^{®} flow cytometry. Gene expression is measured in samples from four donors by qRT-PCR. Cytokine production is measured in samples from at least three donors by ELISA after three days of culture with anti-CD2, anti-CD3, anti-CD28 activation beads.

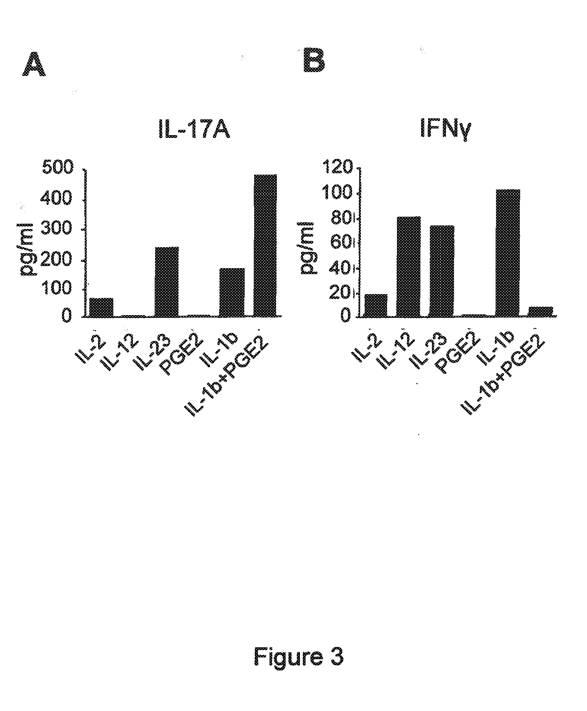

FIGS. 3A and 3B show the expression of IL-17A and IFN-γ, respectively, from human peripheral blood mononuclear CD4⁺ T lymphocytes cultured with IL-2, IL-12, IL-23, PGE2, IL-1β, or (IL-1β + PGE2).

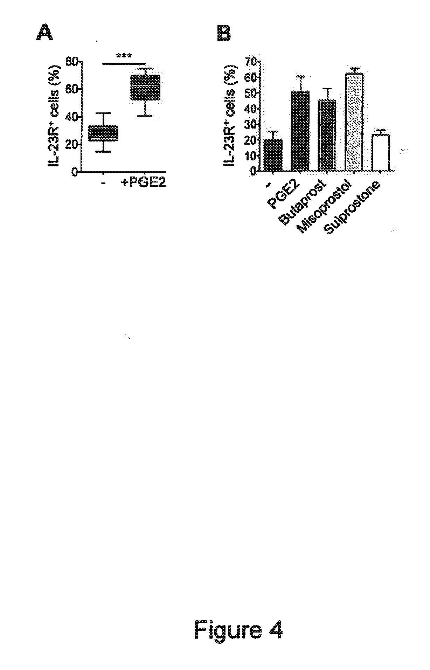

FIGS. 4A and 4B present results obtained in experiments in which naïve human CD4⁺ T cells are activated with T cell activation beads and cultured in the presence or absence of PGE2 or specific EP receptors agonists. FIG. 4A shows the percentage of IL-23R⁺ cells as a function of PGE2, as measured by flow cytometric quantification of IL-23R in T cells restimulated for 48 hours, based on the results of five independent experiments (mean + s.e.m., ***P<0.001). FIG. 4B shows results for cells treated with PGE2 or the specific EP receptor agonists butaprost (EP2 selective agonist), misoprostol (EP4, EP3>EP1>EP2 nonselective agonist), and sulprostone (EP1/EP3 selective agonist), based on the results of of two independent experiments (mean + s.e.m.).

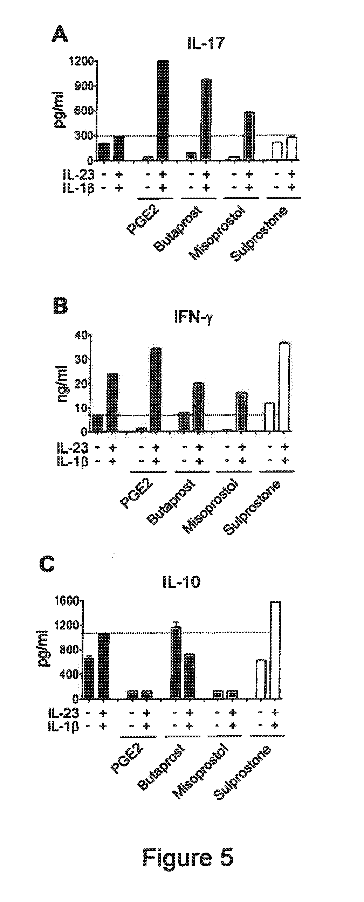

FIGS. 5A, 5B and 5C present results obtained in naïve human CD4⁺ T cells activated with T cell activation beads and cultured in the presence or absence of IL-23 and IL-1β, with or without PGE2 or the EP receptors agonists butaprost, misoprostol, and sulprostone. Data in FIGS. 5A - 5C reflect IL-17A, IFN-γ and IL-10 production in cell-free supernatants ofT cells restimulated for 48 hours, respectively. Results in each figure are representative of two independent experiments.

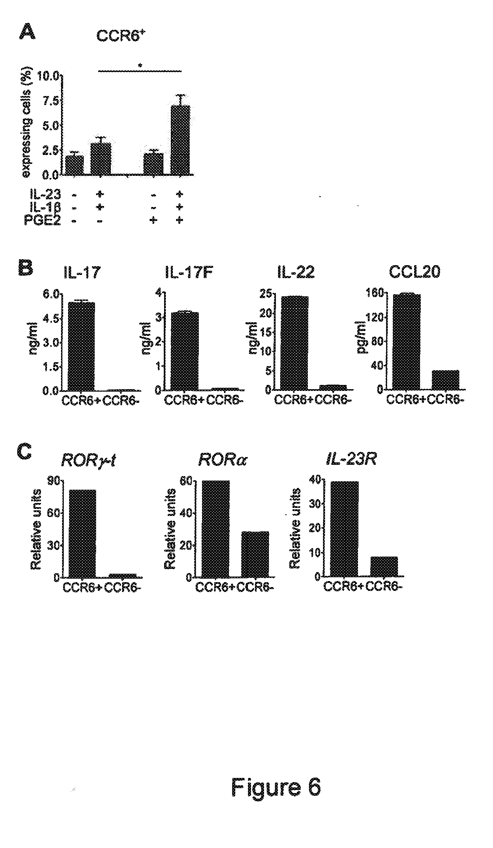

FIGS. 6A, 6B and 6C present results obtained with naïve human CD4⁺ T cells cultured as described with reference to FIGS. 5A - 5C. FIG. 6A shows flow cytometric quantification of CCR6⁺ in T cells restimulated for 48 h. ***P,0.001. Results represent mean + s.e.m. of nine independent experiments. For data presented in FIGS. 6B and 6C, naïve T cells are cultured in the presence of IL-1β, IL-23, and PGE2. After reactivation, CD4⁺CCR6⁺ and CD4⁺CCR6⁻ T cells are sorted and cultured for seven days in the presence of IL-2. FIG. 6B shows production of IL-17A, IL-17F, IL-22, and CCL20 in cell-free supernatants ofT cells restimulated for 24 hours. FIG. 6C shows real-time RT-PCR analysis of ROR-γt, ROR-α, and IL-23R expression in T cells restimulated 24 hours. Results in each of FIGS. 6B and 6C reflect the results of four independent experiments.

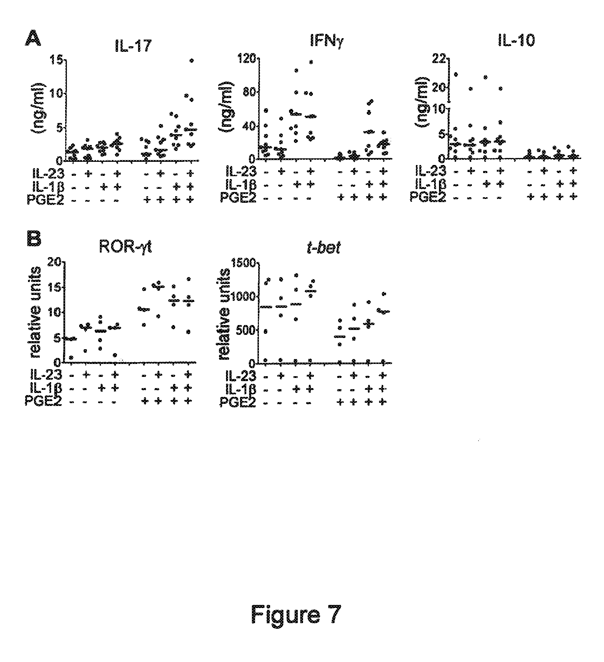

FIGS. 7A and 7B present results obtained with human memory CD4⁺ T cells activated and cultured for three days in the presence of IL-23, IL-1β, and/or PGE2. FIG. 7A shows IL-17A, IFN-γ, and IL-10 production, as indicated, in cell-free supernatants. Results from nine independent donors are shown. FIG. 7B shows the results of real-time RT-PCR of ROR-γt and T-bet gene expression. Results from four different donors are shown. Horizontal lines represent medians.

### DETAILED DESCRIPTION

All references cited herein are incorporated by reference to the same extent as if each individual publication, database entry (e.g. Genbank sequences or GeneID entries), patent application, or patent, was specifically and individually indicated to be incorporated by reference. Citation of the references herein is not intended as an admission that any of the foregoing is pertinent prior art, nor does it constitute any admission as to the contents or date of these publications or documents.

### I. Definitions

As used herein, including the appended claims, the singular forms of words such as "a," "an," and "the," include their corresponding plural references unless the context clearly dictates otherwise.

"Mature" proteins or "mature form" of a protein refers to the sequence after removal of the signal sequence from the amino terminus. Protein sequences provided herein (e.g. by reference to genetic database accession numbers) will typically be proproteins or precursor protein sequences that include a -20 amino acid N-terminal signal sequence that is not present in the mature form of the protein.

Unless otherwise indicated, IL-17, as used herein, refers to IL-17A.

Unless otherwise indicated, proteins referred to herein, such as cytokines, are the human forms of the proteins.

"Proliferative activity" encompasses an activity that promotes, that is necessary for, or that is specifically associated with, e.g., normal cell division, as well as cancer, tumors, dysplasia, cell transformation, metastasis, and angiogenesis.

"Administration" and "treatment," as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. "Administration" and "treatment" can refer, e.g., to therapeutic, pharmacokinetic, diagnostic, research, and experimental methods. Treatment of a cell encompasses contact of a reagent to the cell, as well as contact of a reagent to a fluid, where the fluid is in contact with the cell. "Administration" and "treatment" also means *in vitro* and *ex vivo* treatments, e.g., of a cell, by a reagent, diagnostic, binding composition, or by another cell. "Treatment," as it applies to a human, veterinary, or research subject, refers to therapeutic treatment, prophylactic or preventative measures, to research and diagnostic applications. "Treatment" as it applies to a human, veterinary, or research subject, or cell, tissue, or organ, encompasses contact of an agent with animal subject, a cell, tissue, physiological compartment, or physiological fluid. "Treatment of a cell" also encompasses situations where the agent contacts a target, such as IL-23 receptor, e.g., in the fluid phase or colloidal phase, but also situations where the agonist or antagonist does not contact the cell or the receptor.

"Treat" or "Treating" may also refer to administration of a therapeutic agent, such as a composition described herein, internally or externally to a patient in need of the therapeutic agent. Typically, the agent is administered in an amount effective to prevent or alleviate one or more disease symptoms, or one or more adverse effects of treatment with a different therapeutic agent, whether by preventing the development of, inducing the regression of, or inhibiting the progression of such symptom(s) or adverse effect(s) by any clinically measurable degree. The amount of a therapeutic agent that is effective to alleviate any particular disease symptom or adverse effect (also referred to as the "therapeutically effective amount") may vary according to factors such as the disease state, age, and weight of the patient, the ability of the therapeutic agent to elicit a desired response in the patient, the overall health of the patient, the method, route and dose of administration, and the severity of side affects. *See, e.g.,* U.S. Pat. No. 5,888,530.

Whether a disease symptom or adverse effect has been alleviated can be assessed by any clinical measurement typically used by physicians or other skilled healthcare providers to assess the severity or progression status of that symptom or adverse effect. When a therapeutic agent is administered to a patient who has active disease, a therapeutically effective amount will typically result in a reduction of the measured symptom by at least 5%, usually by at least 10%, more usually at least 20%, most usually at least 30%, preferably at least 40%, more preferably at least 50%, most preferably at least 60%, ideally at least 70%, more ideally at least 80%, and most ideally at least 90%. *See,* e.g., Maynard et al. (1996) A Handbook of SOPs for Good Clinical Practice, Interpharm Press, Boca Raton, FL; Dent (2001) Good Laboratory and Good Clinical Practice, Urch Publ., London, UK.

While an embodiment of the present invention (e.g., a treatment method or article of manufacture) may not be effective in preventing or alleviating the target disease symptom(s) or adverse effect(s) in every patient, it should alleviate such symptom(s) or effect(s) in a statistically significant number of patients as determined by any statistical test known in the art such as the Student's t-test, the chi²-test, the U-test according to Mann and Whitney, the Kruskal-Wallis test (H-test), Jonckheere-Terpstra-test and the Wilcoxon-test.

An "antagonist," as used herein, is any agent that reduces the activity of a targeted molecule. Specifically, an antagonist of a cytokine (such as IL-23, IL-17A, IL-17F, TNF-α or IL-1β) is an agent that reduces the biological activity of that cytokine, for example by blocking binding of the cytokine to its receptor or otherwise reducing its activity (e.g. as measured in a bioassay). As such, an antagonist of a cytokine includes any agent that reduces signaling by the cytokine, and thus may include agents that bind to the cytokine itself, and also agents that binds to its receptor(s). An antagonist further includes an agent that reduces the expression of a cytokine or its receptor, including but not limited to nucleic acid-based antagonists, such as antisense nucleic acids and siRNA. *See, e.g.,* Arenz and Schepers (2003) Naturwissenschaften 90:345-359; Sazani and Kole (2003) J. Clin. Invest. 112:481-486; Pirollo et al. (2003) Pharmacol. Therapeutics 99:55-77; Wang et al. (2003) Antisense Nucl. Acid Drug Devel. 13:169-189.

An agent that acts as an antagonist of one cytokine (e.g. IL-23) may optionally act as an antagonist of another cytokine, e.g. in a bispecific antibody. As such, a method involving "combination therapy" and a composition for such combination therapy need not comprise more than one therapeutic agent.

Cytokine antagonists include, but are not limited to, antagonistic antibodies, peptides, peptide-mimetics, polypeptides, and small molecules that bind to a cytokine (or any of its subunits) or its functional receptor (or any of its subunits) in a manner that interferes with cytokine signal transduction and downstream activity. Examples of peptide and polypeptide antagonists include truncated versions or fragments of the cytokine receptor (e.g., soluble extracellular domains) that bind to the cytokine in a manner that either reduces the amount of cytokine available to bind to its functional receptor or otherwise prevents the cytokine from binding to its functional receptor.

The inhibitory effect of an antagonist can be measured by routine techniques. For example, to assess the inhibitory effect on cytokine-induced activity, human cells expressing a functional receptor for a cytokine are treated with the cytokine and the expression of genes known to be activated or inhibited by that cytokine is measured in the presence or absence of a potential antagonist. Antagonists useful in the present invention inhibit the targeted activity by at least 25%, preferably by at least 50%, more preferably by at least 75%, and most preferably by at least 90%, when compared to a suitable control.

"Binding compound" refers to a molecule, small molecule, macromolecule, polypeptide, antibody or fragment or analogue thereof, or soluble receptor, capable of binding to a target. "Binding compound" also may refer to a complex ofmolecules, e.g., a non-covalent complex, to an ionized molecule, and to a covalently or non-covalently modified molecule, e.g., modified by phosphorylation, acylation, cross-linking, cyclization, or limited cleavage, that is capable of binding to a target. When used with reference to antibodies, the term "binding compound" refers to both antibodies and antigen binding fragments thereof. "Binding" refers to an association of the binding composition with a target where the association results in reduction in the normal Brownian motion of the binding composition, in cases where the binding composition can be dissolved or suspended in solution. "Binding composition" refers to a binding compound in combination with a stabilizer, excipient, salt, buffer, solvent, or additive.

"Small molecule" is defined as a molecule with a molecular weight that is less than 10 kDa, typically less than 2 kDa, and preferably less than 1 kDa. Small molecules include, but are not limited to, inorganic molecules, organic molecules, organic molecules containing an inorganic component, molecules comprising a radioactive atom, synthetic molecules, peptide mimetics, and antibody mimetics. As a therapeutic, a small molecule may be more permeable to cells, less susceptible to degradation, and less apt to elicit an immune response than large molecules. Small molecules, such as peptide mimetics of antibodies and cytokines, as well as small molecule toxins are described. *See, e.g.,* Casset et al. (2003) Biochem. Biophys. Res. Commun. 307:198-205; Muyldermans (2001) J. Biotechnol. 74:277-302; Li (2000) Nat. Biotechnol. 18:1251-1256; Apostolopoulos et al. (2002) Curr. Med. Chem. 9:411-420; Monfardini et al. (2002) Curr. Pharm. Des. 8:2185-2199; Domingues et al. (1999) Nat. Struct. Biol. 6:652-656; Sato and Sone (2003) Biochem. J. 371:603-608; U.S. Patent No. 6,326,482.

As used herein, the term "antibody" refers to any form of antibody that exhibits the desired biological activity. Thus, it is used in the broadest sense and specifically covers monoclonal antibodies (including full length monoclonal antibodies), polyclonal antibodies, multispecific antibodies (e.g., bispecific antibodies), chimeric antibodies, humanized antibodies, fully human antibodies, etc. so long as they exhibit the desired biological activity. Biological activities of antagonist antibodies include inhibiting binding of a cytokine to its receptor, or inhibiting cytokine-induced signaling through a receptor.

Antibodies used in the present invention will usually bind with at least a K_{d} of about 10⁻³ M, more usually at least 10⁻⁶ M, typically at least 10⁻⁷ M, more typically at least 10⁻⁸ M, preferably at least about 10⁻⁹ M, and more preferably at least 10⁻¹⁰ M, and most preferably at least 10⁻¹¹ M. *See, e.g.,* Presta et al. (2001) Thromb. Haemost. 85:379-389; Yang et al. (2001) Crit. Rev. Oncol. Hematol. 38:17-23; Carnahan et al. (2003) Clin. Cancer Res. (Suppl.) 9:3982s-3990s.

"Specifically" or "selectively" binds, when referring to a ligand/receptor, antibody/antigen, or other binding pair, indicates a binding reaction that is determinative of the presence of the protein in a heterogeneous population of proteins and other biologics. Thus, under designated conditions, a specified ligand binds to a particular receptor and does not bind in a significant amount to other proteins present in the sample. As used herein, an antibody is said to bind specifically to a polypeptide comprising a given sequence (e.g. IL-23p19) if it binds to polypeptides comprising the sequence of IL-23p19 but does not bind to proteins lacking the sequence of IL-23p19. For example, an antibody that specifically binds to a polypeptide comprising IL-23p19 may bind to a FLAG^{®}-tagged form of IL-23p19 but will not bind to other FLAG^{®}-tagged proteins.

Unless otherwise indicated, an antagonist of IL-23 refers to an IL-23-specific antagonist. Despite their shared cytokine and receptor subunits, an IL-23-specific antagonist does not also antagonize IL-12. IL-23-specific antagonists include agents that bind to IL-23 and/or IL-23 receptor, including but not limited to agents that bind to IL-23p19 and IL-23R. An agent that antagonizes both IL-23 and IL-12 is referred to herein as an "IL-12/IL-23 antagonist." Such IL-12/IL-23 antagonists include, but are not limited to, agents that bind to IL-12p40 and IL-12Rβ1, which are shared subunits.

The antibody, or binding composition derived from the antigen-binding site of an antibody, of the contemplated method binds to its antigen with an affinity that is at least two fold greater, preferably at least ten times greater, more preferably at least 20-times greater, and most preferably at least 100-times greater than the affinity with unrelated antigens. In a preferred embodiment the antibody will have an affinity that is greater than about 10⁹ liters/mol, as determined, e.g., by Scatchard analysis. Munsen et al. (1980) Analyt. Biochem. 107:220-239.

The term "monoclonal antibody," as used herein, refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256: 495, or may be made by recombinant DNA methods (*see, e.g.,* U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Pat. No. 4,816,567; Morrison et al. (1984) Proc. Natl. Acad. Sci. USA 81: 6851-6855.

As used herein, the term "humanized antibody" refers to forms of antibodies that contain sequences from non-human (*e.g.,* murine) antibodies as well as human antibodies. Such antibodies contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. The humanized forms of rodent antibodies will generally comprise the same CDR sequences of the parental rodent antibodies, although certain amino acid substitutions may be included to increase affinity, increase stability of the humanized antibody, or for other reasons.

The term "antibody" also includes "fully human" antibodies, i.e., antibodies that comprise human immunoglobulin protein sequences only. A fully human antibody may contain murine carbohydrate chains if produced in a mouse, in a mouse cell, or in a hybridoma derived from a mouse cell. Similarly, "mouse antibody" or "rat antibody" refer to an antibody that comprises only mouse or rat immunoglobulin sequences, respectively. A fully human antibody may be generated in a human being, in a transgenic animal having human immunoglobulin germline sequences, by phage display or other molecular biological methods. Also, recombinant immunoglobulins may also be made in transgenic mice. *See* Mendez et al. (1997) Nature Genetics 15:146-156. *See also* Abgenix and Medarex technologies.

The antibodies of the present invention also include antibodies with modified (or blocked) Fc regions to provide altered effector functions. See, e.g., U.S. Pat. No. 5,624,821; WO 2003/086310; WO 2005/120571; WO 2006/0057702; Presta (2006) Adv. Drug Delivery Rev. 58:640-656. Such modification can be used to enhance or suppress various reactions of the immune system, with possible beneficial effects in diagnosis and therapy. Alterations of the Fc region include amino acid changes (substitutions, deletions and insertions), glycosylation or deglycosylation, and adding multiple Fc. Changes to the Fc can also alter the half-life of antibodies in therapeutic antibodies, and a longer half-life would result in less frequent dosing, with the concomitant increased convenience and decreased use of material. *See* Presta (2005) J. Allergy Clin. Immunol.116:731 at 734-35.

The antibodies of the present invention also include antibodies with intact Fc regions that provide full effector functions, e.g. antibodies ofisotype IgGl, which induce complement-dependent cytotoxicity (CDC) or antibody dependent cellular cytotoxicity (ADCC) in the a targeted cell.

The antibodies may also be conjugated (e.g., covalently linked) to molecules that improve stability of the antibody during storage or increase the half-life of the antibody *in vivo.* Examples of molecules that increase the half-life are albumin (e.g., human serum albumin) and polyethylene glycol (PEG). Albumin-linked and PEGylated derivatives of antibodies can be prepared using techniques well known in the art. *See, e.g.,* Chapman (2002) Adv. Drug Deliv. Rev. 54:531-545; Anderson and Tomasi (1988) J. Immunol. Methods 109:37-42; Suzuki et al. (1984) Biochim. Biophys. Acta 788:248-255; and Brekke and Sandlie (2003) Nature Rev. 2:52-62.

As used herein, the terms "binding fragment" or "antigen binding fragment" encompass a fragment or a derivative of an antibody that still substantially retains its biological activity, e.g. inhibiting cytokine signaling via the cytokine receptor. The term "antibody fragment" refers to a portion of a full length antibody, generally the antigen binding or variable region thereof. Examples of antibody fragments include Fab, Fab', F(ab')₂, and Fv fragments; diabodies; linear antibodies; single-chain antibody molecules, e.g., sc-Fv; and multispecific antibodies formed from antibody fragments. Typically, a binding fragment or derivative retains at least 10% of its inhibitory activity. Preferably, a binding fragment or derivative retains at least 25%, 50%, 60%, 70%, 80%, 90%, 95%, 99% or 100% (or more) of its inhibitory activity, although any binding fragment with sufficient affinity to exert the desired biological effect will be useful. It is also intended that an antibody binding fragment can include variants having conservative amino acid substitutions that do not substantially alter its biologic activity.

A "Fab fragment" is comprised of one light chain and the C_{H}1 and variable regions of one heavy chain. The heavy chain of a Fab molecule cannot form a disulfide bond with another heavy chain molecule.

An "Fc" region contains two heavy chain fragments comprising the C_{H}1 and C_{H}2 domains of an antibody. The two heavy chain fragments are held together by two or more disulfide bonds and by hydrophobic interactions of the C_{H}3 domains.

A "Fab' fragment" contains one light chain and a portion of one heavy chain that contains the V_{H} domain and the C_{H}1 domain and also the region between the C_{H}1 and C_{H}2 domains, such that an interchain disulfide bond can be formed between the two heavy chains of two Fab' fragments to form a F(ab') ₂ molecule.

A "F(ab')₂ fragment" contains two light chains and two heavy chains containing a portion of the constant region between the C_{H}1 and C_{H}2 domains, such that an interchain disulfide bond is formed between the two heavy chains. A F(ab')₂ fragment thus is composed of two Fab' fragments that are held together by a disulfide bond between the two heavy chains.

The "Fv region" comprises the variable regions from both the heavy and light chains, but lacks the constant regions.

A "single-chain Fv antibody (or "scFv antibody") refers to antibody fragments comprising the V_{H} and V_{L} domains of an antibody, wherein these domains are present in a single polypeptide chain. Generally, the Fv polypeptide further comprises a polypeptide linker between the V_{H} and V_{L} domains which enables the scFv to form the desired structure for antigen binding. For a review of scFv, see Pluckthun (1994) THE PHARMACOLOGY OF MONOCLONAL ANTIBODIES, vol. 113, Rosenburg and Moore eds. Springer-Verlag, New York, pp. 269-315. *See also* WO 88/01649 and U.S. Pat. Nos. 4,946, 778 and 5,260,203. Such scFv polypeptides may optionally be joined with Fc regions to form scFv-Fc constructs. *See, e.g.,* Powers et al. (2001) J. Immunol. Methods 251:123.

A "diabody" is a small antibody fragment with two antigen-binding sites, which fragments comprise a heavy chain variable domain (V_{H}) connected to a light chain variable domain (V_{L}) in the same polypeptide chain (V_{H}-V_{L} or V_{L}-V_{H}). By using a linker that is too short to allow pairing between the two domains on the same chain, the domains are forced to pair with the complementary domains of another chain and create two antigen-binding sites. Diabodies are described more fully in, *e.g.,* EP 404,097; WO 93/11161; and Holliger et al. (1993) Proc. Natl. Acad. Sci. USA 90: 6444-6448. For a review of engineered antibody variants generally see Holliger and Hudson (2005) Nat. Biotechnol. 23:1126-1136.

A "domain antibody fragment" is an immunologically functional immunoglobulin fragment containing only the variable region of a heavy chain or the variable region of a light chain. In some instances, two or more V_{H} regions are covalently joined with a peptide linker to create a bivalent domain antibody fragment. The two V_{H} regions of a bivalent domain antibody fragment may target the same or different antigens.

A "bivalent antibody" comprises two antigen binding sites. In some instances, the two binding sites have the same antigen specificities. However, bivalent antibodies may be bispecific (see below).

The monoclonal antibodies herein also include camelized single domain antibodies. *See, e.g.,* Muyldennans et al. (2001) Trends Biochem. Sci. 26:230; Reichmann et al. (1999) J. Immunol. Methods 231:25; WO 94/04678; WO 94/25591; U.S. Pat. No. 6,005,079). In one embodiment, the present invention provides single domain antibodies comprising two V_{H} domains with modifications such that single domain antibodies are formed.

Bispecific antibodies are also useful in the present methods and compositions. As used herein, the term "bispecific antibody" refers to an antibody, typically a monoclonal antibody, having binding specificities for at least two different antigenic epitopes. In one embodiment, the epitopes are from the same antigen. In another embodiment, the epitopes are from two different antigens. Methods for making bispecific antibodies are known in the art. For example, bispecific antibodies can be produced recombinantly using the co-expression of two immunoglobulin heavy chain/light chain pairs. *See, e.g.,* Milstein et al. (1983) Nature 305: 537-39. Alternatively, bispecific antibodies can be prepared using chemical linkage. *See, e.g.,* Brennan et al. (1985) Science 229:81. Bispecific antibodies include bispecific antibody fragments. *See, e.g.,* Holliger et al. (1993) Proc. Natl. Acad Sci. US.A. 90:6444-48, Gruber et al. (1994) J. Immunol. 152:5368. Potentially bispecific antibody fragments include diabodies, Bis-ScFv, bivalent domain antibody fragments, Fab₂, and even Fab₃ fragments (which may be trispecific) (*see* Holliger and Hudson (2005) Nat. Biotechnol. 23:1126) and Bis-scFv-Fc. Bispecific antibodies also include dual variable domain immunoglobulins, such as those disclosed at U.S. Patent Application Publication No. 2005/0071675.

The antibodies of the present invention also include antibodies or fragments thereof conjugated to cytotoxic payloads, such as cytotoxic agents or radionuclides. Such antibody conjugates may be used in immunotherapy to selectively target and kill cells expressing a target (the antigen for that antibody) on their surface. Exemplary cytotoxic agents include ricin, vinca alkaloid, methotrexate, *Psuedomonas* exotoxin, saporin, diphtheria toxin, cisplatin, doxorubicin, abrin toxin, gelonin and pokeweed antiviral protein. Exemplary radionuclides for use in immunotherapy with the antibodies of the present invention include ¹²⁵I, ¹³¹I, ⁹⁰Y, ⁶⁷Cu, ²¹¹At, ¹⁷⁷Lu, ¹⁴³Pr and ²¹³Bi. *See, e.g.,* U.S. Patent Application Publication No. 2006/0014225.

"Immune condition" or "immune disorder" encompasses, e.g., pathological inflammation, an inflammatory disorder, and an autoimmune disorder or disease. "Immune condition" also refers to infections, persistent infections, and proliferative conditions, such as cancer, tumors, and angiogenesis, including infections, tumors, and cancers that resist eradication by the immune system. "Cancerous condition" includes, e.g., cancer, cancer cells, tumors, angiogenesis, and precancerous conditions such as dysplasia.

"Inflammatory disorder" means a disorder or pathological condition where the pathology results, in whole or in part, from, e.g., a change in number, change in rate of migration, or change in activation, of cells of the immune system. Cells of the immune system include, e.g., T cells, B cells, monocytes or macrophages, antigen presenting cells (APCs), dendritic cells, microglia, NK cells, NKT cells, neutrophils, eosinophils, mast cells, or any other cell specifically associated with the immunology, for example, cytokine-producing endothelial or epithelial cells.

An "IL-17-producing cell" means a T cell that is not a classical TH 1 -type T cell or classical TH2-type T cell, referred to as Th17 cells. Th17 cells are discussed in greater detail at Cua and Kastelein (2006) Nat. Immunol. 7:557-559; Tato and O'Shea (2006) Nature 441:166-168; Iwakura and Ishigame (2006) J. Clin. Invest. 116:1218-1222. "IL-17-producing cell" also means a T cell that expresses a gene or polypeptide of Table 10B of U.S. Patent Application Publication No. 2004/0219150 (e.g., mitogen responsive P-protein; chemokine ligand 2; interleukin-17 (IL-17); transcription factor RAR related; and/or suppressor of cytokine signaling 3), where expression with treatment by an IL-23 agonist is greater than treatment with an IL-12 agonist, where "greater than" is defined as follows. Expression with an IL-23 agonist is ordinarily at least 5-fold greater, typically at least 10-fold greater, more typically at least 15-fold greater, most typically at least 20-fold greater, preferably at least 25-fold greater, and most preferably at least 30-fold greater, than with IL-12 treatment. Expression can be measured, e.g., with treatment of a population of substantially pure IL-17 producing cells. A Th17 response is an immune response in which the activity and/or proliferation of Th17 cells are enhanced, typically coupled with a repressed Th1 response.

Moreover, "IL-17-producing cell" includes a progenitor or precursor cell that is committed, in a pathway of cell development or cell differentiation, to differentiating into an IL-17-producing cell, as defined above. A progenitor or precursor cell to the IL-17 producing cell can be found in a draining lymph node (DLN). Additionally, "IL-17-producing cell" encompasses an IL-17-producing cell, as defined above, that has been, e.g., activated, e.g., by a phorbol ester, ionophore, and/or carcinogen, further differentiated, stored, frozen, desiccated, inactivated, partially degraded, e.g., by apoptosis, proteolysis, or lipid oxidation, or modified, e.g., by recombinant technology.

As used herein, the term "isolated nucleic acid molecule" refers to a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

As used herein, the term "immunomodulatory agent" refers to natural or synthetic agents that suppress or modulate an immune response. The immune response can be a humoral or cellular response. Immunomodulatory agents encompass immunosuppressive or anti-inflammatory agents.

"Immunosuppressive agents," "immunosuppressive drugs," or "immunosuppressants" as used herein are therapeutics that are used in immunosuppressive therapy to inhibit or prevent activity of the immune system. Clinically they are used to prevent the rejection of transplanted organs and tissues (e.g. bone marrow, heart, kidney, liver), and/or in the treatment of autoimmune diseases or diseases that are most likely of autoimmune origin (e.g. rheumatoid arthritis, myasthenia gravis, systemic lupus erythematosus, ulcerative colitis, multiple sclerosis). Immunosuppressive drugs can be classified into four groups: glucocorticoids cytostatics; antibodies (including Biological Response Modifiers or DMARDs); drugs acting on immunophilins; other drugs, including known chemotherpeutic agents used in the treatment of proliferative disorders. For multiple sclerosis, in particular, the antibodies of the present invention can be administered in conjunction with a new class of myelin binding protein-like therapeutics, known as copaxones.

"Anti-inflammatory agents" or "anti-inflammatory drugs", is used to represent both steroidal and non-steroidal therapeutics. Steroids, also known as corticosteroids, are drugs that closely resemble cortisol, a hormone produced naturally by adrenal glands. Steroids are used as the main treatment for certain inflammatory conditions, such as: Systemic vasculitis (inflammation of blood vessels); and Myositis (inflammation of muscle). Steroids might also be used selectively to treat inflammatory conditions such as: rheumatoid arthritis (chronic inflammatory arthritis occurring in joints on both sides of the body); systemic lupus erythematosus (a generalized disease caused by abnormal immune system function); Sjögren's syndrome (chronic disorder that causes dry eyes and a dry mouth).

Non-steroidal anti-inflammatory drugs, usually abbreviated to NSAIDs, are drugs with analgesic, antipyretic and anti-inflammatory effects - they reduce pain, fever and inflammation. The term "non-steroidal" is used to distinguish these drugs from steroids, which (amongst a broad range of other effects) have a similar eicosanoid-depressing, anti-inflammatory action. NSAIDs are generally indicated for the symptomatic relief of the following conditions: rheumatoid arthritis; osteoarthritis; inflammatory arthropathies (e.g. ankylosing spondylitis, psoriatic arthritis, Reiter's syndrome); acute gout; dysmenorrhoea; metastatic bone pain; headache and migraine; postoperative pain; mild-to-moderate pain due to inflammation and tissue injury; pyrexia; and renal colic. NSAIDs include salicylates, arlyalknoic acids, 2-arylpropionic acids (profess), N-arylanthranilic acids (fenamic acids), oxicams, coxibs, and sulphonanilides.

"Interleukin-17" (or "IL-17," or "IL-17A"), unless otherwise indicated, means a protein consisting of one or two polypeptide chains, with each chain consisting essentially of the sequence of the mature form of human IL-17A as described in any of NCBI Protein Sequence Database Accession Numbers NP_002181, AAH67505, AAH67503, AAH67504, AAH66251, AAH66252 or naturally occurring variants thereof. "Interleukin-17F" (or "IL-17F") means a protein consisting of one or two polypeptides chains, with each chain consisting essentially of the sequence of the mature form of human IL-17F as described at NCBI Protein Sequence Database Accession Number NP_443104.1.

"IL-17R" or "IL-17RA" means a single polypeptide chain consisting essentially of the sequence of the mature form of human IL-17RA as described in WO 96/29408 or in any of NCBI Protein Sequence Database Accession Numbers: NP_055154, Q96F46, CAJ86450, or naturally occurring variants of these sequences.

"IL-17RC" means a single polypeptide chain consisting essentially of the sequence of the mature form of human IL-17RC as described in WO 02/38764 or in any of NCBI Protein Sequence Database Accession Numbers NP_703191, NP_703190 and NP_116121, or naturally occurring variants of these sequences.

"IL-17 receptor" means either IL-17RA, IL-17RC, or other IL-17 receptor subunit, or a dimeric complex of two of these receptor subunits (either homodimeric or heterodimeric).

"Interleukin-23 (or "IL-23") means a protein consisting of two polypeptide subunits, p19 and p40. The sequence of the p19 subunit (also known as IL-23p19, IL23A) is provided at any of NCBI Protein Sequence Database Accession Numbers NP_057668, AAH67511, AAH66267, AAH66268, AAH66269, AAH667512, AAH67513 or naturally occurring variants of these sequences. The sequence of the p40 subunit (also known as IL-12p40, IL12B) as described in any of NCBI Protein Sequence Database Accession Numbers NP_002178, P29460, AAG32620, AAH74723, AAH67502, AAH67499, AAH67498, AAH67501 or naturally occurring variants of these sequences.

"Interleukin-23R" or "IL-23R" means a single polypeptide chain consisting essentially of the sequence of the mature form of human IL-23R as described in NCBI Protein Sequence Database Accession Number NP_653302 (IL23R, Gene ID: 149233) or naturally occurring variants thereof. Additional IL-23R sequence variants are disclosed at WO 01/23556 and WO 02/29060.

"Interleukin-12Rβ1" or "IL-12Rβ1" means a single polypeptide chain consisting essentially of the sequence of the mature form of human IL-12Rβ1 as described in NCBI Protein Sequence Database Accession Numbers NP_714912, NP_005526 (IL12RB1, Gene ID: 35p4) or naturally occurring variants thereof.

"TNF-α" means a single polypeptide chain consisting essentially of the sequence of the mature form of human TNF-α as described in NCBI Protein Sequence Database Accession Number NP_000585 (TNF, Gene ID: 7124) or naturally occurring variants thereof.

"TNF-α receptor" refers to the mature form of either tumor necrosis factor receptor 1 precursor (TNFRSF1A, Gene ID: 7132) as described in NCBI Protein Sequence Database Accession Number NP_001056), or tumor necrosis factor receptor 2 precursor (TNFRSF1B, Gene ID No: 7133) as described in NCBI Protein Sequence Database Accession Number NP_001057), or naturally occurring variants thereof.

"Interleukin-1β" or "IL-1β" means a single polypeptide chain consisting essentially of the sequence of the mature form of human IL-1β as described in NCBI Protein Sequence Database Accession Number NP_000567 (IL1B, Gene ID: 3553) or naturally occurring variants thereof.

"Interleukin-1β receptor" means a single polypeptide chain consisting essentially of the sequence of the mature form of human IL-1β receptor type I precursor, as described in NCBI Protein Sequence Database Accession Number NP_000868 (IL1R1, Gene ID: 3554) or naturally occurring variants thereof.

"CD161" refers to the NK cell surface antigen disclosed in U.S. Pat. No. 5,965,401. The protein is also known as, e.g., KLRB1 and NKRP1A. *See* GeneID 3820. The amino acid sequence for CD161 is available at GenBank (NCBI) accession number NP_002249.

"PGE2" refers to prostaglandin E2. "PGE2 antagonist" refers to any agent that inhibits the activity of PGE2 by any mechanism, such as blocking the synthesis of PGE2 or the binding of PGE2 to its receptor(s).

The phrase "consists essentially of," or variations such as "consist essentially of" or "consisting essentially of," as used throughout the specification and claims, indicate the inclusion of any recited elements or group of elements, and the optional inclusion of other elements, of similar or different nature than the recited elements, that do not materially change the basic or novel properties of the specified dosage regimen, method, or composition. As a non-limiting example, a binding compound that consists essentially of a recited amino acid sequence may also include one or more amino acids, including substitutions of one or more amino acid residues, that do not materially affect the properties of the binding compound.

### II. Combination Therapy for Immune Disorders

The present invention provides compositions and methods for treatment of subject having immune disorders, such as autoimmune disease, involving combination therapy with an antagonist of IL-23 and an antagonist of at least one other pro-inflammatory cytokine, e.g. IL-17A, IL-17F, TNF-α and IL-1β.

A number of cytokines have a role in the pathology or repair of neurological disorders. IL-6, IL-17, interferon-gamma (IFNgamma, IFN-γ), and granulocyte colony-stimulating factor (GM-CSF) have been associated with multiple sclerosis. Matusevicius et al. (1999) Multiple Sclerosis 5:101-104; Lock et al. (2002) Nature Med. 8:500-508. IL-1α, IL-1β, and transforming growth factor-beta 1 (TGF-β1) play a role in ALS, Parkinson's disease, and Alzheimer's disease. Hoozemans et al. (2001) Exp. Gerontol. 36:559-570; Griffin and Mrak (2002) J. Leukocyte Biol. 72:233-238; Ilzecka et al. (2002) Cytokine 20:239-243. TNF-α, IL-1β, IL-6, IL-8, IFN-γ, and IL-17 appear to modulate response to brain ischemia. *See, e.g.,* Kostulas et al. (1999) Stroke 30:2174-2179; Li et al. (2001) J. Neuroimmunol. 116:5-14. Vascular endothelial cell growth factor (VEGF) is associated with ALS. Cleveland and Rothstein (2001) Nature 2:806-819.

Inflammatory bowel disorders, e.g., Crohn's disease, ulcerative colitis, celiac disease, and irritable bowel syndrome, are mediated by cells of the immune system and by cytokines. For example, Crohn's disease is associated with increased IL-12 and IFNγ, while ulcerative colitis is associated with increased IL-5, IL-13, and TGF-β. IL-17 expression may also increase in Crohn's disease and ulcerative colitis. *See, e.g.,* Podolsky (2002) New Engl. J. Med. 347:417-429; Bouma and Strober (2003) Nat. Rev. Immunol. 3:521-533; Bhan et al. (1999) Immunol. Rev. 169:195-207; Hanauer (1996) New Engl. J. Med. 334:841-848; Green (2003) The Lancet 362:383-391; McManus (2003) New Engl. J. Med. 348:2573-2574; Horwitz and Fisher (2001) New Engl. J. Med. 344:1846-1850; Andoh et al. (2002) Int. J. Mol. Med. 10:631-634; Nielsen et al. (2003) Scand. J. Gastroenterol. 38:180-185; Fujino et al. (2003) Gut 52:65-70.

Inflammatory diseases of the skin, joints, CNS, as well as proliferative disorders elicit similar immune responses, thus IL-23/IL-23R blockade should prove useful in treatment of a number of immune mediated inflammatory disorders, such as inflammatory bowel disease, Crohn's disease, ulcerative colitis, rheumatoid arthritis, psoriatic arthritis, psoriasis, atopic dermatitis, multiple sclerosis, type I diabetes, and SLE. IL-23/IL-23R inhibitors will also find use in treatment of proliferative disorders, e.g. cancer and tumors. Descriptions ofIL-23 in these various disorders can be found in the following published PCT applications: WO 04/081190; WO 04/071517; WO 00/53631; and WO 01/18051. IL-23/IL-23R inhibitors may also find use in treatment of infections, including chronic infections, such as bacterial, mycobacterial, viral and fungal infections.

IL-23 plays an important role in the development of Th17 cells, which have recently been implicated in the pathogenesis of a number of autoimmune diseases. The role of Th17 cells in the pathogenesis of several autoimmune inflammatory disorders suggests that IL-23 is "upstream" of the various pro-inflammatory effector cytokines, such as IL-17, TNF-α, and IL-1β. IL-23 is said to be "upstream" in that it is primarily involved in early events in the onset of pathogenic immune response, rather than the subsequent effector (acute) phase of the response. *See, e.g.,* Thakker et al. (2007) J. Immunol. 178:2589. These later, acute phase cytokines generate the localized inflammation that gives rise to the signs and symptoms associated with a flare-up of the disease. IL-23, in contrast, appears to be more important for the long-term survival and proliferation of Th17 cells. It is this difference in the biological roles for various cytokines in the aberrant inflammatory response that is exploited in some embodiments of the methods and compositions of the present invention.

In some embodiments, the methods and compositions of the present invention involve administration of an antagonist of an acute phase cytokine (e.g. IL-17A, IL-17F, TNF-α, and IL-1β) to a subject having an immune disorder, such as an autoimmune disease, early in treatment to rapidly reduce the signs and symptoms of the disease. Antagonists of acute phase cytokines are referred to herein for convenience as "acute phase therapeutic agents." In one embodiment, more than one acute phase therapeutic agent is used. Typically, a subject will be experiencing a flare-up when treatment with an acute phase therapeutic agent is started. This initial treatment is combined with administration of an antagonist ofIL-23, which is optionally started at the same time, or during, treatment with the acute phase therapeutic agent(s), and continues after administration of the acute phase therapeutic agent(s) has been discontinued. Whereas the acute phase therapeutic agent is intended to provide relatively rapid relief of signs and symptoms, IL-23 antagonists are intended primarily to reduce the likelihood of recurrence of the signs and symptoms in a future flare-up. Although there may no longer be a need for the acute phase therapeutic agent after resolution of one, two or all of the symptoms of disease, treatment with an IL-23 antagonist may be required even in the asymptomatic patient to prevent relapse. The specific combination of therapeutic agents, and the respective timing of their administration, provide a comprehensive disease management protocol for autoimmune disorder, particularly those of a relapsing-remitting character.

Antagonists useful in the present invention include a soluble receptor comprising the extracellular domain of a functional receptor for IL-17A, IL-17F, TNF-α, IL-1β or IL-23. Soluble receptors can be prepared and used according to standard methods. See, e.g., Jones et al. (2002) Biochim. Biophys. Acta 1592:251-263; Prudhomme et al. (2001) Expert Opinion Biol. Ther. 1:359-373; Fernandez-Botran (1999) Crit. Rev. Clin. Lab Sci. 36:165-224.

Preferred IL-23 antagonists are antibodies that bind to, and inhibit the activity of, any of IL-23, IL-23p19, IL-12p40, IL-23R, IL-12Rβ1, and an IL-23R/IL-12Rβ1 complex. Another preferred IL-23 antagonist is an IL-23 binding polypeptide which consists essentially of the extracellular domain of IL-23F, e.g., amino acids 1-353 of GenBankAAM44229, or a fragment thereof.

IL-23 antagonists of the present invention, such as inhibitory IL-23p19 and IL-23R-specific antibodies, can inhibit the biological activity of IL-23 in any manner, including but not limited to reducing production of IL-1β and TNF-α by peritoneal macrophages and IL-17 by Th17 cells. *See* Langrish et al. (2004) Immunol. Rev. 202:96-105. IL-23 antagonists will also be able to inhibit the gene expression of IL-17A, IL-17F, CCL7, CCL17, CCL20, CCL22, CCR1, and GM-CSF. *See* Langrish et al. (2005) J. Exp. Med. 201:233-240. IL-23 antagonists will also block the ability of IL-23 to enhance proliferation or survival of Th17 cells. Cua and Kastelein (2006) Nat. Immunol. 7:557-559. The inhibitory activity ofIL-23 antagonists will be useful in the treatment of inflammatory, autoimmune, and proliferative disorders. Examples of such disorders are described in PCT patent application publications WO 04/081190; WO 04/071517; WO 00/53631; and WO 01/18051. Exemplary assays for the determination of IL-23 antagonist activity are provided at Examples 2 and 3, *infra.* Exemplary antibodies to IL-23p19 are disclosed at PCT patent application publication WO 2007/024846, U.S. Patent Application Publication Nos. 2007/0009526 and 2007/0048315, and in commonly-assigned, co-pending U.S. Patent Application Nos. 60/891,413 and 60/891,409. Antagonists of IL-23 also include aptamers, as disclosed at U.S. Patent Application No. 2006/0193821. Other nucleic acid inhibitors of IL-23 include antisense polynucleotides and siRNA molecules, e.g. as disclosed at U.S. Patent Application Publication No. 2005/0261219. Additional anti-IL-23 antibodies are disclosed at U.S. Patent Application Publication No. 2006/0067936. Compounds that reduce the production of IL-23 are disclosed at U.S. Patent Application Publication No. 2006/0135518.

Preferred IL-17 antagonists for use in the present invention are antibodies that specifically bind to, and inhibit the activity of, any of IL-17, IL-17RA, IL-17RC, and a heteromeric complex comprising IL-17RA and IL-17RC. More preferably, the target of the IL-17 antagonist is IL-17 or IL-17RA. Particularly preferred IL-17 antagonists specifically bind to, and inhibit the activity of IL-17. Exemplary antibodies to IL-17A are disclosed at WO 2006/013107 and WO 2008/021156.

Preferred TNF-α antagonists for use in the present invention are antibodies that specifically bind to, and inhibit the activity of, TNF-α or its receptor. Exemplary anti-TNF-α antibodies are available, e.g., as infliximab, etanercept and adalimumab.

Preferred IL-1β antagonists for use in the present invention are antibodies that specifically bind to, and inhibit the activity of, IL-1β or its receptor. Exemplary antibodies to IL-1β include CDP 484 (a PEGylated anti-IL-1β fragment), and antibodies disclosed at U.S. Patent Application Publication No. 2003/0124617. IL-1β antagonists also include IL-1 receptor antagonist anakinra (Kineret^{®} IL-1 receptor antagonist). Such agents have found use in the treatment of rheumatoid arthritis. Gabay and Arend (1998) Springer Semin. Immunopathol. 20:229.

Another preferred IL-23 antagonist for use in the present invention is a bispecific antibody, or bispecific antibody fragment, which also antagonizes the activity of a cytokine selected from the group consisting of IL-17A, IL-17F, TNF-α, and IL-1β. Such bispecific antagonists specifically bind to, and inhibit the activity of, the following combinations: IL-17 and IL-23; IL-17 and IL-23p19; IL-17 and IL-12p40; IL-17 and an IL-23R/IL-12RB1 complex; IL-17 and IL-23R; IL-17 and IL-12RB1; IL-17RA and IL-23; IL-17RA and IL-23p19; IL-17RA and IL-12p40; IL-17RA and an IL-23R/IL-12RB1 complex; IL-17RA and IL-23R; IL-17RA and IL-12RB1; IL-17RC and IL-23; IL-17RC and IL-23p19; IL-17RC and IL-12p40; IL-17RC and an IL-23R/IL-12RB1 complex; IL-17RC and IL-23R; IL-17RC and IL-12RB1; an IL-17RA/IL-17RC complex and IL-23; an IL-17RA/IL-17RC complex and IL-23p19; an IL-17RA/IL-17RC complex and IL-12p40; an IL-17RA/IL-17RC complex and an IL-23R/IL-12RB1 complex; an IL-17RA/IL-17RC complex and IL-23R; and an IL-17RA/IL-17RC complex and IL-12RB1. Preferred combinations targeted by bispecific antibodies used in the present invention are: IL-17 and IL-23, e.g. IL-17 and IL-23p19; IL-17RA and IL-23, e.g. IL-17RA and IL-23p19. A particularly preferred bispecific antibody specifically binds to, and inhibits the activity of, each of IL-17 and IL-23p19.

Bispecific antibodies that antagonize both IL-17 and IL-23 activity can be produced by any technique known in the art. For example, bispecific antibodies can be produced recombinantly using the co-expression of two immunoglobulin heavy chain/light chain pairs. *See, e.g.,* Milstein et al. (1983) Nature 305: 537-39. Alternatively, bispecific antibodies can be prepared using chemical linkage. *See, e.g.,* Brennan et al. (1985) Science 229: 81. These bifunctional antibodies can also be prepared by disulfide exchange, production of hybrid-hybridomas (quadromas), by transcription and translation to produce a single polypeptide chain embodying a bispecific antibody, or transcription and translation to produce more than one polypeptide chain that can associate covalently to produce a bispecific antibody. The contemplated bispecific antibody can also be made entirely by chemical synthesis. The bispecific antibody may comprise two different variable regions, two different constant regions, a variable region and a constant region, or other variations.

Although the specific examples of IL-23-antagonist bispecific antibodies listed in the preceding two paragraphs relate to antagonism ofIL-17 and not to antagonism of TNF-α or IL-1β, analogous bispecific antibodies that antagonize TNF-α and IL-1β are also within the scope of the present invention. Such IL-23-antagonist bispecific antibodies may bind, e.g., to TNF-α or IL-1β or any of their respective receptors or receptor subunits.

Bispecific reagents will also find use in the methods and compositions of the present invention. In one embodiment, the invention relates to combination therapy using a bispecific reagent that binds to two targets selected from the group consisting of IL-23 (e.g. p19 and/or p40), IL-23R (e.g. IL-23R and/or IL-12Rβ1), IL-17A, IL-17F, IL-1 7 receptor (e.g. IL-17RA and/or IL-17RC), TNF-α, IL-1β, TNF-α receptor (and soluble fragments thereof), and IL-1 receptor (and soluble fragments thereof). In some embodiments the bispecific reagent comprises a complex of a first polypeptide derived from antigen binding site of an antibody, and a second polypeptide comprising a soluble receptor fragment. In some embodiments, the first and second polypeptides are fusion proteins comprising antibody Fc domains, e.g. human heavy chain IgG1 or IgG2a. In still further embodiments the Fc domains are modified using a "knobs into holes" approach to promote efficient heterodimeric association of the two polypeptide chains to form a bispecific reagent, rather than the monospecific (bivalent) form that might otherwise result from homodimer formation. *See* Zhu et al. (1997) Protein Sci.6:781*.*

Antibody antagonists for use in the invention may be prepared by any method known in the art for preparing antibodies. The preparation of monoclonal, polyclonal, and humanized antibodies is described in Sheperd and Dean (eds.) (2000) Monoclonal Antibodies, Oxford Univ. Press, New York, NY; Kontermann and Dubel (eds.) (2001) Antibody Engineering, Springer-Verlag, New York; Harlow and Lane (1988) Antibodies A Laboratory Manual, Cold Spring Harbor Laboratory Press, Cold Spring Harbor, NY, pp. 139-243; Carpenter et al. (2000) J. Immunol. 165:6205; He et al. (1998) J. Immunol. 160:1029; Tang et al. (1999) J. Biol. Chem. 274:27371-27378; Baca et al. (1997) J. Biol. Chem. 272:10678-10684; Chothia et al. (1989) Nature 342:877-883; Foote and Winter (1992) J. Mol. Biol. 224:487-499; and U.S. Pat. No. 6,329,511 issued to Vasquez et al.

Any antigenic form of the desired target can be used to generate antibodies, which can be screened for those having the desired antagonizing activity. The eliciting antigen may be a peptide containing a single epitope or multiple epitopes, or it may be the entire protein alone or in combination with one or more immunogenicity enhancing agents known in the art. To improve the immunogenicity of an antigenic peptide, the peptide may be conjugated to a carrier protein. The antigen may also be an isolated full-length protein, a cell surface protein (e.g. immunizing with cells transfected with at least a portion of the antigen), or a soluble protein (*e*.*g*. immunizing with only the extracellular domain portion of the protein). The antigen may be expressed by a genetically modified cell, in which the DNA encoding the antigen is genomic or non-genomic (*e*.*g*. on a plasmid).

A peptide consisting essentially of a region of predicted high antigenicity can be used for antibody generation. For example, regions of high antigenicity of human p19 occur at amino acids 16-28; 57-87; 110-114; 136-154; and 182-186 of GenBank AAQ89442 (gi:37183284) and regions of high antigenicity of human IL-23R occur at amino acids 22-33; 57-63; 68-74; 101-112; 117-133; 164-177; 244-264; 294-302; 315-326; 347-354; 444-473; 510-530; and 554-558 of GenBank AAM44229 (gi: 21239252), as determined by analysis with a Parker plot using Vector NTI® Suite (Informax, Inc, Bethesda, MD).

Any suitable method of immunization can be used. Such methods can include use of adjuvants, other immunostimulants, repeated booster immunizations, and the use of one or more immunization routes. Immunization can also be performed by DNA vector immunization. Wang et al. (1997) Virology 228:278-284. Alternatively, animals can be immunized with cells bearing the antigen of interest, which may provide superior antibody generation than immunization with purified antigen. Kaithamana et al. (1999) J. Immunol. 163:5157-5164.

Preferred antibody antagonists are monoclonal antibodies, which may be obtained by a variety of techniques familiar to skilled artisans. Methods for generating monoclonal antibodies are generally described in Stites et al. (eds.) (1982) BASIC AND CLINICAL IMMUNOLOGY (4th ed.) Lange Medical Publications, Los Altos, CA, and references cited therein; Harlow and Lane (1988) ANTIBODIES: A LABORATORY MANUAL CSH Press; Goding (1986) MONOCLONAL ANTIBODIES: PRINCIPLES AND PRACTICE (2d ed.) Academic Press, New York, NY. Typically, splenocytes isolated from an immunized mammalian host are immortalized, commonly by fusion with a myeloma cell to produce a hybridoma. *See* Kohler and Milstein (1976) Eur. J. Immunol. 6:511-519; Meyaard et al. (1997) Immunity 7:283-290; Wright et al. (2000) Immunity 13:233-242; Preston et al. (1997) Eur. J. Immunol. 27:1911-1918. Alternative methods of immortalization include transformation with Epstein Barr Virus, oncogenes, or retroviruses, or other methods known in the art. *See, e.g.,* Doyle et al. (eds. 1994 and periodic supplements) CELL AND TISSUE CULTURE: LABORATORY PROCEDURES, John Wiley and Sons, New York, NY. Colonies arising from single immortalized cells are screened for production of antibodies of the desired specificity, affinity and inhibiting activity using suitable binding and biological assays. For example, antibody to target binding properties can be measured, e.g., by surface plasmon resonance (Karlsson et al. (1991) J. Immunol. Methods 145:229-240; Neri et al. (1997) Nat. Biotechnol. 15:1271-1275; Jonsson et al. (1991) Biotechniques 11:620-627) or by competition ELISA (Friguet et al. (1985) J. Immunol. Methods 77:305-319; Hubble (1997) Immunol. Today 18:305-306).

Alternatively, one may isolate DNA sequences that encode a monoclonal antibody or a binding fragment thereof by screening a DNA library from human B cells. See e.g., Huse et al. (1989) Science 246:1275-1281. Other suitable techniques involve screening phage antibody display libraries. Huse et al. (1989) Science 246:1275-1281; Ward et al. (1989) Nature 341:544-546; Clackson et al. (1991) Nature 352: 624-628; Marks et al. (1991) J. Mol. Biol. 222: 581-597; Presta (2005) J. Allergy Clin. Immunol. 116:731.

Preferred monoclonal antibodies for use in the present invention include "chimeric" antibodies (immunoglobulins) in which the variable domain is from the parental antibody generated in an experimental mammalian animal, such as a rat or mouse, and the constant domains are obtained from a human antibody, so that the resulting chimeric antibody will be less likely to elicit an adverse immune response in a human subject than the parental mammalian antibody. More preferably, a monoclonal antibody used in the present invention is a "humanized antibody", in which all or substantially all of the hypervariable loops (e.g., the complementarity determining regions or CDRs) in the variable domains correspond to those of a non-human immunoglobulin, and all or substantially all of the framework (FR) regions in the variable domains are those of a human immunoglobulin sequence. A particularly preferred monoclonal antibody for use in the present invention is a "fully human antibody", e.g., an antibody that comprises human immunoglobulin protein sequences only. A fully human antibody may contain carbohydrate chains from the cell species in which it is produced, e.g., if produced in a mouse, in a mouse cell, or in a hybridoma derived from a mouse cell, a fully human antibody will typically contain murine carbohydrate chains.

Bispecific reagents of the present invention may be particularly useful in situations where simultaneous binding of a single reagent (e.g. an antibody) to two different antigens provides added specificity and/or toxicity, such as cell surface antigens. For example, bispecific reagents, such as a bispecific antibodies, may also be generated with agents that bind to cell surface proteins associated with a pathogenic T cell subset, such as pathogenic Th17 cells. In one example, such cell surface proteins are IL-23R and CD161 (also referred to as NK cell surface antigen, KLRB1 (GeneID 3820), and NKRP1A). *See also* U.S. Pat. Nos. 5,965,401 and 5,770,387. The amino acid sequence for CD161 is available at GenBank (NCBI) database under accession number NP_002249. As demonstrated herein (see Example 4 and FIGS. 1 and 2), the presence of CD161 on the surface of memory T cells (CD4⁺/CD45RO⁺/CD45RA⁻) correlates with IL-17 production and pathogenicity. Pathogenic Th17 cells are also known to express IL-23R. Bispecific reagents that bind to both CD161 and IL-23R would be expected to be highly selective for only the most pathogenic T cells. Such specific reagents will find use in diagnosis and monitoring of subjects, including those undergoing treatment, as a tool to measure of the presence and localization of highly pathogenic Th17 cells. These reagents will also find use in therapeutic applications, where they can specifically promote killing of the pathogenic target cells, i.e. those cells expressing both CD161 and IL-23R. The reagents, e.g. antibodies comprising a human IgG1 constant domain, may promote ADCC (antigendependent cellular cytotoxicity) dependent killing of pathogenic Th17 cells. The reagents may also be used to deliver a toxic payload to such pathogenic Th17 cells, e.g. a radionuclide or other toxin.

Additional bispecific reagents that may find use in treatment of diseases caused by pathogenic Th17 cells include reagents directed to two or more cell surface molecules found on these cells, wherein specificity of the reagent for the combination of said two or more cell surface molecules renders the reagent more specific for the pathogenic cells. Such enhanced specificity may be helpful in reducing side effects caused by undesired effects on non-target cells expressing any one of the cell surface molecules. For example, a bispecific reagent may be directed to CD161 and any other cell surface marker associated with pathogenic Th17 cells, including but not limited to CD4, CD45RO, CCR4, CCR6, integrin-β7, EP2, EP4, IL-1R1, or TNF-α. Alternatively, a bispecific reagent may be directed to any two of the following cell surface proteins: CD161, CD4, CD45RO, CCR4, CCR6, integrin-β7, EP2, EP4, IL-1R1, and TNF-α.

### Role of PGE2, IL-23 and IL-1β in Generation of Pathogenic Human Th17 Cells

Recent publications involving studies in mice have demonstrated that IL-6 and TGF-β are necessary and sufficient to generate Th17 cells, and that IL-23 is important in promoting the maintenance and survival of these cells. Veldhoen et al. (2006) Immunity 24:179-189; Dong (2006) Nat. Rev. Immunol. 6(4):329-333. These results, however, have not been repeated in a human system, leaving open the question of the importance of the IL-6 and TGF-β in the generation of Th17 cells, and thus in human autoimmune and proliferative disease.

Applicants have found that these IL-6 / TGF-β-driven mouse Th17 cells secrete not only IL-17A, but also very high levels of the immunosuppressive cytokine IL-10. Whereas IL-23-driven Th17 cells are able to induce experimental autoimmune encephalomyelitis (EAE) in a passive transfer model in mice (Langrish et al. (2005) J. Exp. Med. 201:233), IL-6 / TGF-β-driven mouse Th17 cells are non-pathogenic. Applicants' further experiments in mice now demonstrate that prostaglandin E2 (PGE2), in combination with IL-1β, drives the formation of a novel mouse CD4⁺ Th17 population secreting high levels of IL-17A, but not IL-10. These effects are consistent with the observation (by quantitative PCR) that CD4+ T cells express the IL-1β and PGE2 receptor subunits IL-1R1, IL-1Racp, EP2 and EP4. These same murine IL-1β / PGE2-driven The17 cells exhibit increased expression of the transcription factor FOXP3.

These results in mice led Applicants to search for a similar pathogenic Th17 lineage in humans. Although culture in the presence of IL-6 and TGF-β did not promote the development of human Th17 cells, Applicants have found that PGE2 acts synergistically with EL-1β to promote formation of a pathogenic subset of human Th17 cells. These pathogenic Th17 cells produce high levels of IL-17A and very low levels of IFN-γ, indicative of a high degree of polarization toward a Th17 (IL-17-producing) phenotype and away from a Th1 (IFN-γ producing) phenotype. Data are presented at FIGS. 3A - 3B. *See also* Example 5. These cells also express high levels of IL-17F, and are likely to be involved in the pathogenesis of human autoimmune diseases such as multiple sclerosis (MS), Crohn's disease (CD) and rheumatoid arthritis (RA). Further data (FIGS. 4-6) bolster the conclusion that PGE2 plays a role in Th17 biology.

Prostaglandins, and in particular prostaglandin E2 (PGE2), play an important role in the regulation of the inflammatory response. PGE2, a key mediator of pyrexia, hyperalgesia, and arterial dilation, increases bloodflow to inflamed tissues and, together with enhanced microvascular permeability, results in edema. Prostaglandin synthesis inhibitors such as cyclooxygenase inhibitors are used clinically as effective anti-inflammatory agents. However, PGE2 can also exert anti-inflammatory properties, and is a key negative regulator of neutrophil, monocyte, and lymphocyte function, particularly Th1 cells. Harris et al. (2002) Trends Immunol. 23:144. This apparent paradox has puzzled many investigators for decades. The interplay among PGE2, IL-23, and IL-1β biology may now reveal the solution to this paradox. The literature demonstrates that IL-23 and the IL-23-dependent Th17 population ofT helper cells play essential roles in chronic inflammation and autoimmunity. Chen et al. (2007) Arthritis Rheum. 56:2936; Cua et al. (2003) Nature 421:744; Langrish et al. (2005) J. Exp. Med. 201:233; Murphy et al. (2003) J. Exp. Med. 198:1951; Wilson et al. (2007) Nature Immunol. 8:950. Using a dendritic-cell free culture system, the results disclosed herein show here that PGE2, in the presence of IL-1β and IL-23 promotes the differentiation and pro-inflammatory function of Th17 cells. PGE2 acts directly on naïve human T cells and upregulates IL-23 receptor expression via prostaglandin receptor EP2 and EP4-mediated signaling. Furthermore, PGE2 synergizes with IL-1β and IL-23 to drive ROR-γt, IL-17, and CCR6 expression, consistent with the reported Th17 phenotype. While enhancing Th17 cytokine expression, PGE2 inhibits IL-10 production. Hence, the combination of inflammatory cytokines and non-cytokine immunomodulators, such as PGE2, present during differentiation determines the ultimate phenotype of Th17 cells. These findings highlight the role of the inflammatory microenvironment as a crucial factor for Th17 cell development and regulation.

As mentioned *supra,* PGE2 exposure increases expression of the transcription factor FOXP3 in mice, and similar results have been reported in human CD4+ T cells. Baratelli et al. (2005) J. Immunol. 175:1483; Mahic et al. (2006) J. Immunol. 177:246. Without intending to be limited by theory, it is possible that PGE2 plays the same role in generation of Th17 cells in humans that TGF-β plays in mice. Regardless of the mechanism of action, PGE2 appears to be necessary for the generation of pathogenic human Th17 cells.

PGE2 has previously been shown to induce production of IL-23 and IL-1β from immature bone marrow-derived dendritic cells, suggesting a pro-inflammatory role for PGE2, and a potential role in autoimmune diseases such as rheumatoid arthritis. Sheibanie et al. (2004) FASEB J. 18: 1318. PGE2 has been shown to have effects in murine models of inflammatory bowel disease and rheumatoid arthritis (collagen induced arthritis) via effects on the IL-23/IL-17 pathway. Sheibanie et al. (2007) J. Immunol. 178:8138; Sheibanie et al. (2007) Arthritis Rheum. 56:2608. *See also* Jefford et al. (2003) Blood 102:1753. These effects have been attributed to PGE2 actions on innate cells, since PGE2 enhances production of IL-23 and IL-1β in macrophages and dendritic cells, while downregulating IL-12 production. Sheibanie et al. (2004) FASEB J. 18:1318. The results presented herein, however, show that PGE2 is directly involved in Th17 development

The fact that pathogenic human Th17 cells can be created *in vitro* by treatment of CD4⁺ T cells with PGE2 and IL-1β, or PGE2 and IL-23, provides an improved method of generating pathogenic human Th17 cells *in vitro,* which cells will find use in basic biomedical research and in drug screening. Potential therapeutic compounds can be screened for their ability to prevent the development of, maintenance of, or block the pathogenic effects of, such pathogenic human Th17 cells in *vitro*.

In light of the important roles played by PGE2, IL-1β and IL-23 in the formation and maintenance of pathogenic Th17 cells, it is also likely that combination therapy targeting two or more of these molecules will be useful in the treatment of autoimmune or proliferative disorders. Antagonists of PGE2, IL-1β and IL-23 include agents that block the biological activities of such molecules in promoting Th17 cell development and maintenance, and thus include antagonists that bind either to the molecules themselves or to their receptors (or subunits thereof). Antagonists also include agents that reduce the activity of any of these molecules, such as small molecule inhibitors. Antagonists also include agents that reduce the expression of IL-1β or IL-23, or the proteins (e.g. enzymes) involved in the synthesis of PGE2. Antagonists may include antibodies or antigen binding fragments thereof, nucleic acid inhibitors (such as siRNA or antisense oligonucleotides), soluble receptor fragments, small molecules, etc.

Exemplary methods of combination therapy to prevent the formation of pathogenic Th17 cells in humans include use of an antagonist of PGE2, in combination with an antagonist of and IL-1β or an antagonist of IL-23. Exemplary antagonists of PGE2 include antagonists of cyclooxygenase (COX) and other enzymes involved in PGE2 synthesis. Exemplary COX inhibitors include aspirin, indomethacin, diclofenac, ibuprofen, naproxen, diflunisal, etodolac, fenoprofen, flurbiprofen, ketoprofen, ketorolac, mefenamic acid, meloxicam, nabumetone, oxaprozin, piroxicam, salsalate, sulindac, and tolmetin, and also include the COX-2-specific inhibitors celecoxib, valdecoxib, lumiracoxib and rofecoxib. COX-2 inhibitors have been suggested for the treatment of experimental autoimmune neuritis (EAN) and experimental autoimmune anterior uveitis (EAAU) in rats, and for treatment of experimental autoimmune encephalomyelitis (EAE), an animal model for MS. *See* Miyamoto et al. (2002) Muscle Nerve 25:280; Bora et al. (2005) Ocul. Immunol. Inflamm. 13:183; and Ni et al. (2007) J. Neuroimmunol. 186:94, respectively. Celecoxib has been suggested for the treatment of multiple sclerosis based on data obtained in an EAE model in mice. Miyamoto et al. (2006) Brain 129:1984.

Antagonists of PGE2 also include antagonists of any of the enzymes involved in specifically in the synthesis of PGE2, including PGE2 synthases (PGESs) such as PGES-2 (Per-Johan Jakobsson et al. (1999) Proc. Natl. Acad. Sci. (U.S.A.) 96:7220) and PGES-1 (U.S. Pat. No. 7,169,580). Specific inhibition of such PGE2-specific synthetic enzymes would be expected to have the advantage of altering PGE2 levels without affecting the levels of other prostaglandins, with concomitant reduction of undesired side effects. Such specific inhibitors include small molecules, antagonistic antibodies or antigen binding fragments thereof, or nucleic acid antagonists such as siRNA or antisense nucleic acids.

Antagonists of PGE2 also include antagonists of the relevant receptors that are expressed on the surface of CD4+ T cells, i.e. EP2 and EP4. These two receptors are referred to herein, collectively, as EP2/4. EP2/4 have been proposed as therapeutic targets for treatment of rheumatoid arthritis. Akaogi et al. (2006) Endocr. Metab. Immune Disord. Drug Targets 6:383. Exemplary antagonists of EP2 and EP4 include antagonistic antibodies or antigen binding fragments thereof, and AH6809 and AH23848. *See, e.g.,* Mahic et al. (2006) J. Immunol. 177:246. Exemplary antagonists of EP2 and EP4 also include nucleic acid antagonists, such as siRNA or antisense nucleic acids. An exemplary EP4 antagonist is disclosed at WO 2000/016760. EP2 is further described at GeneID PTGER2 in the NCBI Gene database, and the protein sequence is available at GenBank Ref. NP_000947.2. EP4 is further described at GeneID PTGER4, and the protein sequence is available at GenBank Ref. NP_000949.1.

Antagonists of EL-1β include antagonists of IL-1β, and also the IL-1 receptor antagonist (IL-1Ra, anakinra), and antagonists of the receptor subunits IL-1R1 and IL-1Racp. Elimination of IL-1R1 in knockout mice has been shown to abrogate induction of Th17 cells and also to significantly lower the incidence of EAE in wild type mice, suggesting a role for IL-1 functions in the formation of Th17 cells and autoimmune disease. Sutton et al. (2006) J. Exp. Med. 203:1685.

Antagonists ofIL-23 include antagonists of IL-23, such as antagonists of the p19 and p40 subunits, and antagonists of the receptor subunits IL-23R and IL-12Rβ1. In preferred embodiments the antagonists are IL-23-specific in that they are directed to the IL-23-specific subunits p19 and IL-23R. Exemplary engineered antibodies to IL-23p19 are disclosed in commonly-assigned U.S. Provisional Patent Application Nos. 60/891,409 and 60/891,413 (both filed 23 February 2007), in U.S. Patent Application Publication Nos. 2007/0009526 and 2007/0048315, and in International Patent Publication Nos. WO 2007/076524, WO 2007/024846 and WO 2007/147019. Antibodies specific for IL-23p40 are disclosed at U.S. Patent No. 7,247,711.

Exemplary combination therapy regimens include, but are not limited to, antagonists of PGE2 and IL-1β, antagonists of EP2/4 and IL-1R1, or antagonists of EP2/4 and IL-23R. In some cases it may be preferable to target both targets at the same time in the same cells, e.g. via bispecific agents. Such combination therapy may effectively block the development and differentiation of pathogenic human Th17 cells, thereby inhibiting human autoimmune and proliferative disorders. In preferred embodiments, the two or more antagonists are antagonists of different mechanistic pathways, rather than antagonists of different parts of the same pathway. In other embodiments at least one of the inhibitors binds to a cell surface receptor rather than a soluble ligand. In some embodiments the two or more antagonists comprise a bifunctional reagent, such as a bispecific antibody or antigen binding fragment thereof, that binds to at least one cell surface receptor. In some embodiments, both targets of the bifunctional reagent of the present invention are cell surface receptors, e.g. EP2/4, and IL-23R or IL-1R1.

### III. Pharmaceutical Formulations, Dosing and Administration

IL-17 antagonists and IL-23 antagonists are typically administered to a patient as a pharmaceutical composition in which the antagonist is admixed with a pharmaceutically acceptable carrier or excipient, see, e.g., Remington's Pharmaceutical Sciences and U.S. Pharmacopeia: National Formulary, Mack Publishing Company, Easton, PA (1984). The pharmaceutical composition may be formulated in any manner suitable for the intended route of administration. Examples of pharmaceutical formulations include lyophilized powders, slurries, aqueous solutions, suspensions and sustained release formulations (see, e.g., Hardman et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis et al. (eds.) (1993) Pharmaceutical Dosage Forms: Parenteral Medications, Marcel Dekker, NY; Lieberman et al. (eds.) (1990) Pharmaceutical Dosage Forms: Tablets, Marcel Dekker, NY; Lieberman et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

The route of administration will depend on the properties of the antagonist or other therapeutic agent used in the pharmaceutical composition. Suitable routes of administration may, for example, include oral, inhalation, rectal, topical, cutaneous, transmucosal, or intestinal administration; parenteral delivery, including intramuscular, subcutaneous, intraarterial or intravenous injection, intramedullary injections, as well as intrathecal, direct intraventricular, intravenous, intraperitoneal, intranasal, or intraocular injections.

Alternately, one may administer the antibody in a local rather than systemic manner, for example, via injection of the antibody directly into an arthritic joint or pathogen-induced lesion characterized by immunopathology, often in a depot or sustained release formulation. Furthermore, one may administer the antibody in a targeted drug delivery system, for example, in a liposome coated with a tissue-specific antibody, targeting, for example, arthritic joint or pathogen-induced lesion characterized by immunopathology. The liposomes will be targeted to and taken up selectively by the afflicted tissue. U.S. Patent App. Pub. No. 2008/0019975 describes induction-maintenance treatment regimens comprising an induction regimen, involving administration of a lower dose of a therapeutic agent by a more invasive and/or localized route, followed by a maintenance regimen, involving administration of a higher dose of the therapeutic agent by a less invasive and/or localized route, e.g. systemically.

Injection of gene transfer vectors into the central nervous system has also been described. *See, e.g.,* Cua et al. (2001) J. Immunol. 166:602-608; Sidman et al. (1983) Biopolymers 22:547-556; Langer et al. (1981) J. Biomed. Mater. Res, 15:167-277; Langer (1982) Chem. Tech. 12:98-105; Epstein et al. (1985) Proc. Natl. Acad. Sci. USA 82:3688-3692; Hwang et al. (1980) Proc. Natl. Acad. Sci. USA 77:4030-4034; U.S. Pat. Nos. 6,350466 and 6,316,024. Such vectors may be of use in embodiments of the present invention in which antisense nucleic acids or siRNA are to be used as cytokine antagonists, specifically in treatment of immune inflammatory disorders of the CNS, such as MS.

The pharmaceutical compositions of the invention may be administered according to any treatment regimen that ameliorates or prevents one or more symptoms of the immune disorder. Selecting the treatment regimen will depend on several composition-dependent and patient-dependent factors, including but not limited to the half-life of the antagonist, the severity of the patient's symptoms, and the type or length of any adverse effects. Preferably, an administration regimen maximizes the amount of therapeutic agent delivered to the patient consistent with an acceptable level of side effects. Guidance in selecting appropriate doses of therapeutic antibodies and small molecules is available. *See,* e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602.

Toxicity and therapeutic efficacy of the antibody compositions, administered alone or in combination with an immunosuppressive agent, can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio of LD₅₀ to ED₅₀. Antibodies exhibiting high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration.

Biological antagonists such as antibodies may be provided by continuous infusion, or by doses at intervals of, e.g., once per day, once per week, or 2 to 7 times per week, once every other week, or once per month. A total weekly dose is generally at least 0.05 µg/kg, 0.2 µg/kg, 0.5 µg/kg, 1 µg/kg, 10 µg/kg, 100 µg/kg, 0.2 mg/kg, 1.0 µg/kg, 2.0 mg/kg, 10 mg/kg, 25 mg/kg, 50 mg/kg body weight or more. *See, e.g.*, Yang et al. (2003) New Engl. J. Med. 349:427-434; Herold et al. (2002) New Engl. J. Med. 346:1692-1698; Liu et al. (1999) J. Neurol. Neurosurg. Psych. 67:451-456; Portielji et al. (20003) Cancer Immunol. Immunother. 52:133-144. The desired dose of a small molecule therapeutic, e.g., a peptide mimetic, natural product, or organic chemical, is about the same as for an antibody or polypeptide, on a moles/kg basis.

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced. Preferably, a biologic that will be used is substantially derived from the same species as the animal targeted for treatment (e.g. a humanized antibody for treatment of human subjects), thereby minimizing any immune response to the reagent.

Treatment regimens using antagonists of IL-17 or other acute phase cytokines along with IL-23 antagonists will typically be determined by the treating physician and will take into account the patient's age, medical history, disease symptoms, and tolerance for different types of medications and dosing regimens. Generally the treatment regimen is designed to suppress the overly aggressive immune system, allowing the body to eventually re-regulate itself, with the result often being that after the patient has been kept on systemic medications to suppress the inappropriate immune response for a finite length of time (for example, one year), medication can then be tapered and stopped without recurrence of the autoimmune attack. Sometimes resumption of the attack does occur, in which case the patient must be re-treated.

Thus, in some cases, the physician may prescribe the patient a certain number of doses of the antagonist to be taken over a prescribed time period, after which therapy with the antagonist is discontinued. Preferably, after an initial treatment period in which one or more of the acute symptoms of the disease disappear, the physician will continue the antagonist therapy for some period of time, in which the amount and/or frequency of antagonist administered is gradually reduced before treatment is stopped.

The present invention also contemplates treatment regimens in which an IL-17 antagonist or other acute phase cytokine antagonist is used in combination with an IL-23 antagonist. (Although the discussion that follows refers only to IL-17, the invention relates, mutatis mutandis, to other acute phase cytokines, such as TNF-α and IL-1β.) Such regimens may be especially useful in treating the acute phase of immune disorder, in which the IL-17 antagonist inhibits the activity of existing Th17 cells, while the IL-23 antagonist prevents the generation of new Th17 cells. Such combination therapy may provide effective treatment of an immune disorder using a lower dose of the IL-17 antagonist and/or administering the IL-17 antagonist for a shorter period of time. As symptoms ameliorate, therapy with IL-17 antagonist is preferably discontinued, while administration of the IL-23 antagonist is continued to prevent generation of new autoreactive Th17 cells that could lead to recurrence of the disease. The two antagonists may be administered at the same time in a single composition, or in separate compositions. Alternately, the two antagonists may be administered at separate intervals. Different doses of the antagonists may also be used. Similarly, a bispecific antagonist may also be administered during the acute phase and gradually withdrawn, followed by treatment with an IL-23 antagonist to maintain repression of the disease.

The treatment regimen may also include use of other therapeutic agents, to ameliorate one or more symptoms of the immune disorder or to prevent or ameliorate adverse effects from the antagonist therapy. Methods for co-administration or treatment with a second therapeutic agent, e.g., a cytokine, antibody, steroid, chemotherapeutic agent, antibiotic, or radiation, are well known in the art, see, e.g., Hardman et al. (eds.) (2001) Goodman and Gilman's The Pharmacological Basis of Therapeutics, 10th ed., McGraw-Hill, New York, NY; Poole and Peterson (eds.) (2001) Pharmacotherapeutics for Advanced Practice: A Practical Approach, Lippincott, Williams & Wilkins, Phila., PA; Chabner and Longo (eds.) (2001) Cancer Chemotherapy and Biotherapy, Lippincott, Williams & Wilkins, Phila., PA. The pharmaceutical composition of the invention may also contain other immunosuppressive or immunomodulating agents. Suitable immunosuppressive agent can be employed, including but not limited to, anti-inflammatory agents, corticosteroids, dexamethasone, flurometholone, and prednisolone, cyclosporine, tacrolimus (*i*.*e*., FK-506), sirolimus, interferons, soluble cytokine receptors (e.g., sTNRF and sIL-1R), mycophenolate mofetil, 15-deoxyspergualin, thalidomide, glatiramer, azathioprine, leflunomide, cyclophosphamide, chlorambucil, non-steroidal anti-inflammatories such as indomethacin, aspirin, flubiprofen and diclofenac, antimetabolites (e.g., methotrexate, azathioprine), and the like. The pharmaceutical composition can also be employed with other therapeutic modalities such as phototherapy and radiation.

In any of the therapies described herein in which two or more different therapeutic substances are used (e.g., an IL-17 antagonist and an IL-23 antagonist, an IL-17 antagonist and a therapeutic agent that does not antagonize IL-17 or IL-23 activity), it will be understood that the different therapeutic substances are administered in association with each other, that is, they may be administered concurrently in the same pharmaceutical composition, as separate compositions, or the substances may be administered at separate times and in different orders.

### IV. Uses

The present invention provides methods and compositions for treatment of immune disorders, specifically autoimmune disorders that follow a relapsing-remitting pattern. Exemplary diseases include MS, rheumatoid arthritis, psoriatic arthritis, psoriasis, atopic dermatitis, inflammatory bowel disease, Crohn's disease, ulcerative colitis, and type I diabetes. Eptitope spreading may be responsible for the relapsing-remitting nature of many inflammatory autoimmune diseases, in which new epitopes drive the formation of new antigen-specific pathogenic Th17 cells. Interference with IL-23 signaling would stop this process by preventing the generation of new pathogenic The17 cells.

Various other autoimmune disorders may involve "migrating" Th17 cells that cause disease in tissues other than the tissue in which the Th17 cells originally arise. Such diseases include, but are not limited to, psoriatic arthritis, uveitis, juvenile onset arthritis, and multiple sclerosis. IL-23-directed therapies would also be expected to be useful in treatment of such diseases. Pathogenic Th17 cells may be targeted for destruction by systemic therapy while in transit from their tissue of origin.

Th 17 cells can be identified based on their expression of a distinctive pattern of serum biomarkers, including IL-23, IL-17, IL-12p70, IL-12p40, TNF- a, IL-1, IL-6, IL-22, IFN-γ, IL-22, CCL20 (MEP-3α) and CXCL1 (GRO). Measurement of any one of, or any combination of, these biomarkers may be used to assess the role of Th17-cell mediated pathology in a disease, and thus the likely therapeutic efficacy of IL-23-neutralization as therapy. The biomarkers may also be used to monitor disease progress, e.g. during a course of treatment.

The methods and compositions of the present invention may also be used in the treatment of cancers, e.g. tumors, in which an aberrant IL-23-mediated Th17 response promotes inflammation in the vicinity of a tumor, and paradoxically represses IL-12-mediated Th1-type tumor surveillance. *See* WO 2004/081190. Transient suppression of the acute inflammatory response and long-term maintenance of anti-IL-23 therapy may promote recovery of IL-12-mediated Th1 tumor surveillance, and promote tumor eradication.

Methods are provided for the treatment of, e.g., multiple sclerosis (MS), including relapsing-remitting MS and primary progressive MS, Alzheimer's disease, amyotrophic lateral sclerosis (a.k.a. ALS; Lou Gehrig's disease), ischemic brain injury, prion diseases, and HIV-associated dementia. Also provided are methods for treating neuropathic pain, posttraumatic neuropathies, Guillain-Barre syndrome (GBS), peripheral polyneuropathy, and nerve regeneration.

Provided are methods for treating or ameliorating one or more of the following features, symptoms, aspects, manifestations, or signs of multiple sclerosis, or other inflammatory disorder or condition of the nervous system: brain lesions, myelin lesions, demyelination, demyelinated plaques, visual disturbance, loss of balance or coordination, spasticity, sensory disturbances, incontinence, pain, weakness, fatigue, paralysis, cognitive impairment, bradyphrenia, diplopia, optic neuritis, paresthesia, gait ataxia, fatigue, Uhtoff's symptom, neuralgia, aphasia, apraxia, seizures, visual-field loss, dementia, extrapyramidal phenomena, depression, sense of well-being, or other emotional symptoms, chronic progressive myelopathy, and a symptom detected by magnetic resonance imaging (MRI), including gadolinium-enhancing lesions, evoked potential recordings, or examination of cerebrospinal fluid. *See, e.g.,* Kenealy et al. (2003) J. Neuroimmunol. 143:7-12; Noseworthy et al. (2000) New Engl. J. Med. 343:938-952; Miller et al. (2003) New Engl. J. Med. 348:15-23; Chang et al. (2002) New Engl. J. Med. 346:165-173; Bruck and Stadelmann (2003) Neurol. Sci. 24 Suppl.5:S265-S267.

Moreover, the present invention provides methods for treating and diagnosing inflammatory bowel disorders, e.g., Crohn's disease, ulcerative colitis, celiac disease, and irritable bowel syndrome. Provided are methods for treating or ameliorating one or more of the following symptoms, aspects, manifestations, or signs of an inflammatory bowel disorder: malabsorption of food, altered bowel motility, infection, fever, abdominal pain, diarrhea, rectal bleeding, weight loss, signs of malnutrition, perianal disease, abdominal mass, and growth failure, as well as intestinal complications such as stricture, fistulas, toxic megacolon, perforation, and cancer, and including endoscopic findings, such as, friability, aphthous and linear ulcers, cobblestone appearance, pseudopolyps, and rectal involvement and, in addition, anti-yeast antibodies. *See, e.g.,* Podolsky, *supra*; Hanauer, *supra;* Horwitz and Fisher, *sura.*

Also contemplated is treatment of inflammatory disorders such as psoriasis, atopic dermatitis, arthritis, including rheumatoid arthritis, osteoarthritis, and psoriatic arthritis, autoimmune disorders, such as SLE and type I diabetes, autoimmune myocarditis (Sonderegger et al. (2006) Eur. J. Immunol. 36:2844), and proliferative disorders such as cancer. *See, e.g.,* PCT patent application publications WO 04/081190; WO 04/071517; WO 00/53631; and WO 01/18051.

The broad scope of this invention is best understood with reference to the following examples, which are not intended to limit the inventions to the specific embodiments. The specific embodiments described herein are offered by way of example only, and the invention is to be limited by the terms of the appended claims, along with the full scope of equivalents to which such claims are entitled.

### EXAMPLES

### Example I

### General Methods

Standard methods in molecular biology are described. Maniatis et al. (1982) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA. Standard methods also appear in Ausbel et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, *centrifugation,* and crystallization are described. Coligan et al. (2000) Current Protocols in Protein Science, Vol. 1, John Wiley and Sons, Inc., New York. Chemical analysis, chemical modification, post-translational modification, production of fusion proteins, glycosylation of proteins are described. *See, e.g.,* Coligan et al. (2000) Current Protocols in Protein Science, Vol. 2, John Wiley and Sons, Inc., New York; Ausubel et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391. Production, purification, and fragmentation of polyclonal and monoclonal antibodies are described. Coligan et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra.* Standard techniques for characterizing ligand/receptor interactions are available. *See, e.g.,* Coligan et al. (2001) Current Protcols in Immunology, Vol. 4, John Wiley, Inc., New York.

Methods for flow cytometry, including fluorescence activated cell sorting detection systems (FACS^{®}), are available. *See, e.g.,* Owens et al. (1994) Flow Cytometry Principles for Clinical Laboratory Practice, John Wiley and Sons, Hoboken, NJ; Givan (2001) Flow Cytometry, 2nd ed.; Wiley-Liss, Hoboken, NJ; Shapiro (2003) Practical Flow Cytometry, John Wiley and Sons, Hoboken, NJ. Fluorescent reagents suitable for modifying nucleic acids, including nucleic acid primers and probes, polypeptides, and antibodies, for use, e.g., as diagnostic reagents, are available. Molecular Probes (2003) Catalogue, Molecular Probes, Inc., Eugene, OR; Sigma-Aldrich (2003) Catalogue, St. Louis, MO.

Standard methods of histology of the immune system are described. *See,* e.g., Muller-Harmelink (ed.) (1986) Human Thymus: Histopathology and Pathology, Springer Verlag, New York, NY; Hiatt et al. (2000) Color Atlas of Histology, Lippincott, Williams, and Wilkins, Phila, PA; Louis et al. (2002) Basic Histology:Text and Atlas, McGraw-Hill, New York, NY.

Software packages and databases for determining, e.g., antigenic fragments, leader sequences, protein folding, functional domains, glycosylation sites, and sequence alignments, are available. *See, e.g.,* GenBank, Vector NTI^{®} Suite (Informax, Inc, Bethesda, MD); GCG Wisconsin Package (Accelrys, Inc., San Diego, CA); DeCypher® (TimeLogic Corp., Crystal Bay, Nevada); Menne et al. (2000) Bioinformatics 16: 741-742; Menne et al. (2000) Bioinformatics Applications Note 16:741-742; Wren et al. (2002) Comput. Methods Programs Biomed. 68:177-181; von Heijne (1983) Eur. J. Biochem. 133:17-21; von Heijne (1986) Nucleic Acids Res. 14:4683-4690.

### Example 2

### Proliferation Bioassays for the Assessment ofIL-23 Antagonists

The ability of an IL-23 antagonist to biologically neutralize IL-23/EL-23R is assessed by the application of short-term proliferation bioassays that employ cells that express recombinant IL-23 receptors. The transfectant Ba/F3-2.2lo cells proliferate in response to human IL-23 and the response can be inhibited by an IL-23 antagonist. The concentration of IL-23 chosen for the assay is selected to be within the linear region of the dose-response curve, near plateau and above EC50. Proliferation, or lack thereof, is measured by colorimetric means using Alamar Blue, a growth indicator dye based on detection of metabolic activity. The ability of an IL-23 antagonist to neutralize IL-23/IL-23R is assessed by its IC50 value, or concentration of antagonist that induces half-maximal inhibition of IL-23-induced proliferation.

The assay is performed essentially as follows. Ba/F3 transfectants are maintained in RPMI-1640 medium, 10% fetal calf serum, 50 µM 2-mercaptoethanol, 2 mM L-Glutamine, 50 µg/mL penicillin-streptomycin, and 10 ng/mL mouse IL-3. Proliferation bioassays are performed in RPMI-1640 medium, 10% fetal calf serum, 50 µM 2-mercaptoethanol, 2 mM L-Glutamine, and 50 µg/mL penicillin-streptomycin.

Assays are performed in 96-well flat bottom plates (Falcon 3072 or similar) in 150 µL per well. Both IL-23 and IL-23 antagonist are prepared at a series of concentrations, e.g. 1:3 serial dilutions. Titrations of the IL-23 antagonist of interest are pre-incubated with IL-23 prior to addition of the cells. After addition of cells, bioassay plates are incubated in a humidified tissue culture chamber (37°C, 5% CO₂) for 40-48 hr. At the end of the culture time, Alamar Blue (Biosource Cat #DAL1100) is added at 16.5 µL/well and allowed to develop for 5-12 hours. Absorbance is then read at 570 nm and 600 nm (VERSAmax Microplate Reader, Molecular Probes, Eugene, Oregon, USA), and an OD₅₇₀₋₆₀₀ is obtained. Duplicates are run for each sample. Absorbance is plotted against cytokine or antibody concentration using GraphPad Prism^{®} 3.0 software (Graphpad Software Inc., San Diego, California, USA), and IC50 values are determined using nonlinear regression (curve fit) of sigmoidal dose-response.

### Example 3

### Splenocyte Assay for IL-23 Based on IL-17 Production

The biological activity of an IL-23 antagonist of the present invention may be assessed using the splenocyte assay essentially as described in Aggarwal et *al.* (2003) *J. Biol. Chem.* 278:1910 and Stumhofer *et al.* (2006) *Nature Immunol.* 7:937. The splenocyte assay measures the activity of IL-23 in a sample as a level of IL-17 production by murine splenocytes. The inhibitory activity of an IL-23 antagonist is then assessed by determining the concentration of antagonist necessary to reduce the IL-23/IL-23R activity in a given sample by 50% (the IC50). The IC50 as measured by this assay is greater than or equal to the equilibrium dissociation binding constant (K_{d}), i.e. the K_{d} may be equal to or lower than the IC50. As always, lower IC50 and K_{d} values reflect higher activities and affinities.

Briefly, spleens are obtained from 8-12 wk old female C57BL/6J mice (Jackson Laboratories, Bar Harbor, Maine, USA). Spleens are ground, pelleted twice, and filtered through a cell strainer (70 µm nylon). The recovered cells are cultured in 96-well plates (4 X 10⁵ cells/well) in the presence of human IL-23 (10 ng/ml) and mouse-anti-CD3e antibodies (1 µg/ml) (BD Pharmingen, Franklin Lakes, New Jersey, USA), with or without the IL-23 antagonist to be assayed. IL-23 antagonists are added at a series of 3-fold dilutions. Cells are cultured for 72 hours, pelleted, and the supernatant is assayed for IL-17 levels by sandwich ELISA.

IL-17 ELISA is performed as follows. Plates are coated with a capture anti-IL-17 antibody (100 ng/well) overnight at 4°C, washed and blocked. Samples and standards are added and incubated for two hours at room temperature with shaking. Plates are washed, and a biotinylated anti-IL-17 detection antibody (100 ng/well) is added and incubated for one hour at room temperature with shaking. The capture and detection antibodies are different antibodies that both bind to mouse IL-17 but do not cross-block. Plates are washed, and bound detection antibody is detected using streptavidin-HRP (horseradish peroxidase) and TMB (3,3',5,5'-tetramethylbenzidine). The plate is then read at 450-650 nm and the concentration of IL-17 in samples is calculated by comparison with standards.

### Example 4

### CD161 is Expressed on Pathogenic Th17 Cells in Crohn's Disease

The17 cells are implicated in the pathology of numerous autoimmune inflammatory and proliferative disorders. IL-23R is a known cell surface receptor subunit that is expressed by Th17 cells. The experiments described herein indicate that the C-type lectin CD161, which is known to be expressed on human NK and T-cells, is preferentially expressed on pathogenic Th17 cells. Such pathogenic cells may be specifically targeted by therapeutic agents that simultaneously bind to both IL-23R and CD161. In addition, the presence of both IL-23R and CD161 on the surface of these cells provides a convenient means of sorting cells for diagnostic and research purposes.

Colon and peripheral blood (PB) samples are obtained from Crohn's Disease patients. Lamina propria mononuclear cells (LPMC) are prepared from colon samples by dissociation of the epithelial layer of the mucosa, collagenase digestion of the lamina propria, and density gradient centrifugation. Peripheral blood mononuclear cells (PBMC) are isolated from PB by density gradient centrifugation and lysis of red blood cells.

Colon samples from CD patients contain approximately 20-fold more CD161⁺ CD4⁺ memory T cells, as determined by flow cytometry, than colon samples from normal subjects. See FIG. 1A. FACS^{®} flow cytometry purified lamina propria CD161⁺ Thₘₑₘ cells are found to produce 4 to 6-fold more IL-17 than CD161⁻ Thₘₑₘ cells (as measured by ELISA) in both CD and normal samples. *See* FIG. 1B. The combination of increased IL-17 production and increased cell numbers indicate that CD161⁺ Thₘₑₘ cells are a major source of IL-17 in the colon of CD patients. Further FACS^{®} flow cytometry experiments demonstrate that approximately one-third of CD161⁺ Thₘₑₘ cells express IL-23R, and culture of CD161⁺ Thₘₑₘ cells in the presence of IL-23 enhanced IL-17 production approximately 3-fold. Gene expression profiling (FIG. 1C) demonstrates that CD161⁺ Thₘₑₘ cells express higher levels various pro-inflammatory cytokines characteristic of the The17 phenotype (IL-23R, IL-17, and IL-22), but not the Thl-associated cytokine IFN-γ, as compared with CD161⁻ cells.

Analogous experiments with PBMC indicate that circulating CD161⁺ CD4⁺ memory T cells also exhibit gene expression and cytokine production profiles consistent with the Th17 phenotype. FIG. 2A shows that several genes known to be associated with Th17 cells (IL-23RA, ROR-γT and IL-17A) are significantly upregulated in CD161⁺ cells as compared to CD161⁻ cells. FIG. 2B shows that production of the known Th17-associated cytokines IL-17A, IL-22 and IL-17F is significantly greater in CD161⁺ cells as compared to CD161⁻cells.

This Th17 phenotype is increased in Crohn's disease patients. FACS^{®} flow cytometry purified CD161⁺ Thₘₑₘ cells from PBMC express significantly higher levels of IL-17 than CD161⁻ cells, but do not differ in the level of IFN-γ production (both as measure by RT quantitative PCR). FACS^{®} flow cytometry demonstrates that approximately 45% of PBMC CD161⁺ Thₘₑₘ cells from CD patients express IL-23R, as compared with -30% for PBMC CD161⁺ Thₘₑₘ cells from normal subjects, and RT quantitative PCR indicates that IL-23R expression is somewhat higher in CD161⁺ Thₘₑₘ cells compared with CD161⁻ Thₘₑₘ cells in both normal and CD PBMC. PBMC CD161⁺ Thₘₑₘ cells from CD patients also exhibit increased IL-17 production when cultured with IL-23.

Taken together, the results obtained with LPMC and PBMC from CD and normal subjects are consistent with the idea the CD161⁺ Thₘₑₘ cell subset described herein represents the same pathogenic The17 cells that have been previously associated with autoimmune inflammatory disorders.

The ability of pathogenic Th17 cells to migrate from the blood into areas of active inflammation is also investigated. Experiments using PBMC from healthy human donors demonstrate that the percentage of integrin-β7⁺ cells and the percentage of CCR6⁺ cells approximately twice as high in CD161⁺ CD4⁺ memory T cells as in CD161⁻ cells (data not shown). Integrin-β7 in complex with integrin-α4 binds to MAdCAM-1, a tissue-specific endothelial cell adhesion molecule that is critical for lymphocyte homing to the gut. Briskin et al. (1993) Nature 363:461 and Berlin et al. (1993) Cell 74:185. CCR6 is preferentially expressed on CD4⁺ memory T cells and facilitates trafficking to epithelial sites. Liao et al. (1999) J. Immunol. 162:186. Its sole chemokine ligand CCL20 (MIP-3α) is dramatically induced in Crohn's disease inflammation. Kwon et al. (2002) Gut 51:818 and Kaser et al. (2004) J. Clin. Immunol. 24:74. These results demonstrate that the CD161⁺ CD4⁺ memory T cells described herein exhibit the homing and chemokine receptor signature that would be expected for cells involved in gut inflammation.

### Example 5

### IL-1β, IL-23 and PGE2 Synergistically Drive Development of Pathogenic Human Th17 Cells

Methods used in this example are generally as described at Wilson et al. (2007) Nature Immunology 8:950. More specifically, cell culture is performed as follows. Naïve CD4⁺ CD45RO⁻ T cells are isolated and cultured as described previously (Wilson *e al.* (2007), *supra).* Memory CD4⁺ CD45RA⁻ T cells are isolated using the memory T cell isolation kit, human (Miltenyi, Auburn, CA), according to the manufacturer's instructions. Where indicated, 50 ng/ml hIL-23, 50 ng/ml hIL-1β (R&D Systems, Minneapolis, MN), 10 µM PGE2 (Sigma, St. Louis, MO), 10 µM butaprost (EP2 selective agonist), 35 µM misoprostol (EP4, EP3>EP1>EP2 agonist), and/or 10 µM sulprostone (EP1, EP3 agonist, Cayman Chemical, Ann Arbor, MI) are added.

Cell sorting is performed as follows. CD4⁺ CCR6⁺ and CD4⁺CCR6⁺ cell subsets are purified by cell sorting using anti-CCR6 and anti-CD4 antibodies (BD Biosciences, San Diego, CA). Cell sorting is done with a FACS^{®} Aria instrument (BD Biosciences).

For analysis of cell surface proteins, cells are stained with anti-CD4, anti-CD3, anti-CD45RA, anti-CCR6 (BD Biosciences), and/or anti-IL-23R (R&D Systems) antibodies. Data are acquired on a LSR II cytometer and analyzed with FlowJo software (Tree Star, Ashland, OR).

ELISA and electrochemiluminescence assays are performed as described previously *(Wilson et al.* (2007), *supra).* IL-10 ELISA is performed using a kit from R&D Systems.

Real-time quantitative PCR is performed as described previously (Wilson *et al.* (2007), *supra).*

Mann Whitney or One-Way ANOVA (for multiple groups) tests are used for statistical analysis. P values of 0.05 or less are considered significant, and all data are represented as mean+s.e.m.

Experiments are performed to determine the effects of IL-12, IL-23, IL-1β and/or PGE2 on cytokine production. Naïve human PBMC CD4⁺ T lymphocytes are activated with anti-CD2, anti-CD3 and anti-CD28 coated beads and cultured in the presence of IL-2, IL-12, IL-23, PGE2, IL-1β, or the combination of PGE2 and IL-1β, for 10 - 12 days. Cultured T cells are re-stimulated with anti-CD2, anti-CD3 and anti-CD28 coated beads in the presence ofIL-2 for 48h, then cell-free supernatants are assessed for IL-17A and IFN-γ production. The results are presented at FIGS. 3A and 3B, respectively. Cells cultured in the presence of both IL-1β and PGE2 exhibit elevated expression of IL-17A and low expression of IFN-γ.

Further experiments are performed to determine the effect of PGE2, or agonists thereof, on the expression of IL-23R in naïve human CD4⁺ T cells in culture. PGE2 exposure more than doubles the percentage of IL-23R expressing CD4⁺ T cells (FIG. 4A), as does exposure to the EP receptor agonists butaprost and misoprostol, but not sulprostone (FIG. 4B). The fact that butaprost (EP2 specific) and misoprostol (EP4, EP3>EP1>EP2) mimic the effects of PGE2, whereas the EP1/EP3 agonist sulprostone does not, suggests that PGE2 signaling occurs via the EP2 and/or EP4 receptors in mediating its effects on naive human CD4⁺ T cells. In addition, IL-1R1 gene expression is increased in response to PGE2 (data not shown).

PGE2 and EP receptor agonists, together with IL-1β and IL-23, have effects on cytokine expression by naïve human CD4⁺ T cells in culture as shown in FIGS. 5A - 5C. The results suggest that PGE2, together with IL-1β and IL-23, enhances human Th17 cell development via the EP2 and EP4 receptors. EP2 agonist butaprost induces a somewhat greater increase in IL-17 expression than misoprostol. Downregulation of the anti-inflammatory cytokine IL-10 (FIG. 5C) is also consistent with a role for PGE2 in induction of Th17-mediated inflammation. *See also* Jankovic & Trinchieri (2007) Nature Immunol. 8:1281 and McGeachy et al. (2007) Nature Immunol. 8:1390, suggesting that IL-10 restrains the pathogenicity of Th17 cells in mice. Comparison of the results obtained with butaprost and misoprostol suggest that the increase in IL-17A is predominantly mediated by EP2 whereas the decrease in IL-10 is predominantly mediated by EP4.

CCR6 expression, which correlates with Th17 cytokine production (FIGS. 6B and 6C), is also responsive to PGE2, as illustrated at FIG. 6A, which shows an increase in the percentage of naïve human CD4⁺ T cells expressing CCR6 when PGE2 is added to IL-1β and IL-23. *See also* Acosta-Rodriguez et al. (2007) Nature Immunol. 8:639 and Annunziato et al. (2007) J. Exp. Med. 204:1849; Singh et al. (2008) J. Immunol. 180:214. CCR6 is involved in recruitment of pathogenic T cells in experimental autoimmune encephalopathy (EAE), rheumatoid arthritis and psoriasis. Homey et al. (2000) J. Immunol. 164:6621; Kohler et al. (2003) J. Immunol. 170:6298; Ruth et al. (2003) Lab. Invest. 83:579. The results presented in FIGS. 3 - 6 are consistent with a role for PGE2 in promoting the development of pathogenic effector Th17 cells.

Results presented at FIGS. 7A (protein expression) and 7B (gene expression) demonstrate that PGE2 enhances a pathogenic Th17 phenotype in activated memory T cells. Specifically, PGE2 generally promotes increased levels of the IL-17A and expression of ROR-γt, both of which are associated with pathogenic Th17 cells, but it does not promote increased levels of IFN-γ or IL-10, nor does it increase expression ofT-bet. Because activated/memory T cells represent a major cell population in inflamed tissue, and in light of the data presented *supra* with respect to naïve human T cells, the results presented herein suggest that the combination of inflammatory cytokines and non-cytokine immunomodulators present during both T cell differentiation in lymph nodes, and during activation in sites of tissue inflammation, will determine the ultimate phenotype of Th17 cells. Intervention with therapeutic agents, such as antagonists of the inflammatory cytokines and non-cytokine immunomodulators, might therefore be expected to be beneficial when administered both systemically and also when administered locally at (or near) the site of inflammation.
In other definitions of the invention, embodiments are provided as described in the following clauses:
1. A method of treating a subject having cancer or exhibiting a flare up of an autoimmune disease, comprising:
   a) administration of an antagonist of IL-23 in a series of one or more doses over a first time interval; and
   b) administration of an acute phase therapeutic agent, comprising an antagonist of a cytokine selected from the group consisting of IL-1β, TNF-α, IL-17A, and IL-17F in a series of one or more doses over a second time interval.
2. The method of 1 wherein the antagonist of IL-23 is an anti-IL-23p19 antibody or an antigen binding fragment thereof.
3. The method of 1 wherein the antagonist ofIL-23 is an anti-IL-23R antibody or an antigen binding fragment thereof.
4. The method of 1 wherein the acute phase therapeutic agent is an antibody that specifically binds to a cytokine selected from the group consisting of IL-1β, TNF-α, IL-17A, and IL-17F.
5. The method of 1 wherein the acute phase therapeutic agent is an antibody that specifically binds to a receptor for a cytokine selected from the group consisting of IL-1β, TNF-α, IL-17A, and IL-17F.
6. The method of 1 wherein the second time interval ends upon the resolution of at least one symptom of the flare-up of the autoimmune disease.
7. The method of 6 wherein the second time interval ends within 30 days of the resolution of two or more symptoms of the flare-up of the autoimmune disease.
8. The method of 1 wherein:
   a) the second time interval begins substantially at the same time the first time interval begins; and
   b) the second time interval ends before the first time interval ends.
9. The method of I wherein
   c) the second time interval begins before the first time interval begins; and
   d) the second time interval ends before the first time interval ends.
10. The method of 1 wherein the second time interval ends before the first time interval begins.
11. The method of any of 8-10 wherein the second time interval is less than 6 months.
12. The method of any of 8-10 wherein the second time interval is less than 2 months.
13. The method of any of 8-10 wherein the first time interval is more than 1 year.
14. The method of any of 8-10 wherein the first time interval is more than 3 years.
15. The method of either of 4 or 5 wherein the antibody or antigen binding fragment thereof is a humanized or fully human antibody or antigen binding fragment thereof.
16. The method of either of 4 or 5 wherein the antibody or antigen binding fragment thereof is a fragment of a humanized or fully human antibody selected from the group consisting Fab, Fab', Fab'-SH, Fv, scFv, F(ab')₂, and a diabody.
17. The method of either of 4 or 5 wherein the antibody or antigen binding fragment thereof is PEGylated.
18. The method of either of 4 or 5 wherein the antibody or antigen binding fragment thereof is a bispecific antibody or antigen binding fragment thereof.
19. The method of 18 wherein the bispecific antibody binds to:
   e) IL-23 or its receptor; and
   f) a cytokine selected from the group consisting of IL-1β, TNF-α, IL-17A, and IL-17F or a receptor for a cytokine selected from the group consisting of IL-1β, TNF-α, IL-17A, and IL-17F.
20. The method of 19 wherein the bispecific antibody binds to:
   g) IL-23; and
   h) a cytokine selected from the group consisting of IL-1β, TNF-α, IL-17A, and IL-17F.
21. A pharmaceutical composition comprising:
   i) an anti-IL-23p19 antibody or antigen binding fragment thereof; and
   j) an antibody, or antigen binding fragment thereof, that binds to a cytokine selected from the group consisting of IL-1β, TNF-α, IL-17A, and IL-17F.
22. The pharmaceutical composition of 21 comprising a bispecific antibody that binds to:
   k) IL-23p19; and
   l) a cytokine selected from the group consisting of IL-1β, TNF-α, IL-17A, and IL-17F.
23. A pharmaceutical composition comprising the pharmaceutical composition of either of 21 or 22, further comprising a pharmaceutically acceptable carrier or diluent.
24. The pharmaceutical composition of 23, further comprising a steroid or a non-steroidal anti-inflammatory agent.
25. The method of any of 1-20, wherein the subject has a disorder selected from the group consisting of cancer, arthritis, rheumatoid arthritis (RA), psoriasis, inflammatory bowel disease, Crohn's disease, ulcerative colitis, multiple sclerosis (MS), systemic lupus erythematosus (SLE), type I diabetes.
26. The method of any of 1-20, further comprising administering an immunosuppressive or anti-inflammatory agent.
27. The method of 26, wherein the immunosuppressive or anti-inflammatory agent is a steroid or a non-steroidal anti-inflammatory agent.
28. A method of treating a subject having cancer or exhibiting a flare up of an autoimmune disease, comprising administering a binding composition derived from the antigen binding sites of an antibody, wherein the binding composition binds to IL-23R and CD161.
29. A method of treating a subject having an autoimmune or proliferative disorder comprising administering to said subject a composition comprising two or more agents selected from the group consisting of an antagonist of IL-23, an antagonist of IL-1β, and an antagonist of PGE2.
30. The method of 29 wherein the agents are selected from the group consisting of an antagonist of an IL-23 receptor, an antagonist of an IL-1β receptor, and an antagonist of a PGE2 receptor.
31. The method of 29 wherein the composition comprises a bispecific antibody or antigen binding fragment thereof.
32. A composition for treatment of an autoimmune or proliferative disorder comprising two or more agents selected from the group consisting of an antagonist of IL-23, an antagonist of IL-1β, and an antagonist of PGE2.
33. The composition of 32 wherein the agents are selected from the group consisting of an antagonist of an IL-23 receptor, an antagonist of an IL-1β receptor, and an antagonist of a PGE2 receptor.
34. The composition of 32 wherein the composition comprises a bispecific antibody or antigen binding fragment thereof.
35. A method of generating pathogenic Th17 cells in *vitro* comprising culturing T cells *in vitro* in the presence of two or more agents selected from the group consisting of IL-23, IL-1β, and PGE2.
36. A method of screening for compounds for use in the treatment of disorders mediated by pathogenic Th17 cells comprising:
   a) generating pathogenic Th17 cells by the method of 35;
   b) exposing said cells to one or more potential therapeutic compounds; and
   c) evaluating the effect of such compound(s) on said Th17 cells.
37. A method of treating a subject having an autoimmune or proliferative disorder comprising:
   a) administering to said subject a composition comprising two or more agents selected from the group consisting of an antagonist of IL-23, an antagonist of IL-1β and an antagonist of PGE2; and
   b) monitoring the level or activity of said pathogenic Th17 cells during or after said administering.
38. The method of 37, wherein said monitoring is by measurement of the level of expression of two or more cytokines selected from the group consisting of IL-17A, IL-17F, IL-10, IL-22 and IFN-γ.

## Claims

1. An antagonist of IL-23 and an acute phase therapeutic agent for use in a method of treating a subject having cancer or exhibiting a flare up of an autoimmune disease, the method comprising:
a) administration of said antagonist in a series of one or more doses over a first time interval; and
b) administration of said acute phase therapeutic agent in a series of one or more doses over a second time interval;
wherein the acute phase therapeutic agent comprises an antagonist of a cytokine selected from the group consisting of IL-1β, TNF-α, IL-17A, and IL-17F.

2. The antagonist and acute phase therapeutic agent_of Claim 1 wherein the antagonist of IL-23 is:
(a) an anti-IL-23p19 antibody or an antigen binding fragment thereof; or
(b) an anti-IL-23R antibody or an antigen binding fragment thereof

3. The antagonist and acute phase therapeutic agent_of Claim 1 wherein the acute phase therapeutic agent is an antibody that specifically binds to a cytokine, or a receptor for a cytokine, selected from the group consisting of IL-1β, TNF-α, IL-17A, and IL-17F.

4. The antagonist and acute phase therapeutic agent of Claim 1 wherein the second time interval ends upon the resolution of at least one symptom of the flare-up of the autoimmune disease.

5. The antagonist and acute phase therapeutic agent of Claim 1 wherein:
a) the second time interval begins before or substantially at the same time the first time interval begins; and
b) the second time interval ends before the first time interval ends.

6. The antagonist and acute phase therapeutic agent of Claim 1 wherein the second time interval ends before the first time interval begins.

7. The antagonist and acute phase therapeutic agent of Claim 5 or Claim 6 wherein the second time interval is less than 6 months, preferably less than 2 months.

8. The antagonist and acute phase therapeutic agent of Claim 3 wherein the antibody or antigen binding fragment thereof is a humanized or fully human antibody or antigen binding fragment thereof

9. The antagonist and acute phase therapeutic agent of Claim 3 wherein the antibody or antigen binding fragment thereof is a fragment of a humanized or fully human antibody selected from the group consisting Fab, Fab', Fab'-SH, Fv, scFv, F(ab')₂, and a diabody.

10. The antagonist and acute phase therapeutic agent of Claim 3 wherein the antibody or antigen binding fragment thereof is PEGylated.

11. The antagonist and acute phase therapeutic agent of Claim 3 wherein the antibody or antigen binding fragment thereof is a bispecific antibody or antigen binding fragment thereof

12. The antagonist and acute phase therapeutic agent_of Claim 11 wherein the bispecific antibody binds to:
a) IL-23 or its receptor; and
b) a cytokine selected from the group consisting of IL-1β, TNF-α, IL-17A, and IL-17F or a receptor for a cytokine selected from the group consisting of IL-1β, TNF-α, IL-17A, and IL-17F.

13. A pharmaceutical composition comprising:
a) an anti-IL-23p19 antibody or antigen binding fragment thereof; and
b) an antibody, or antigen binding fragment thereof, that binds to a cytokine selected from the group consisting of IL-1β, TNF-α, IL-17A, and IL-17F.

14. The pharmaceutical composition of Claim 13 comprising a bispecific antibody that binds to:
a) IL-23p19; and
b) a cytokine selected from the group consisting of IL-1β, TNF-α, IL-17A, and IL-17F.

15. The antagonist and acute phase therapeutic agent_of any of Claims 1-12, wherein the subject has a disorder selected from the group consisting of cancer, arthritis, rheumatoid arthritis (RA), psoriasis, inflammatory bowel disease, Crohn's disease, ulcerative colitis, multiple sclerosis (MS), systemic lupus erythematosus (SLE), type I diabetes.
